(19) Europäisches Patentamt / European Patent Office / Office européen des brevets

(11) **EP 2 781 602 A1**

(12) **EUROPEAN PATENT APPLICATION**

(43) Date of publication:
24.09.2014 Bulletin 2014/39

(51) Int Cl.:
*C12Q 1/68* $^{(2006.01)}$    *G06F 19/00* $^{(2011.01)}$

(21) Application number: **13001450.9**

(22) Date of filing: **21.03.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Universität Konstanz**
**78464 Konstanz (DE)**

(72) Inventors:
• **Bürkle, Alexander, Prof. Dr.**
**78479 Reichenau (DE)**

• **Junk, Michael, Prof. Dr.**
**78464 Konstanz (DE)**
• **Berthold, Michael, prof. Dr.**
**78464 Konstanz (DE)**
• **Moreno-Villanueva, María, Dr.**
**78464 Konstanz (DE)**

(74) Representative: **Müller-Boré & Partner**
**Patentanwälte PartG mbB**
**Friedenheimer Brücke 21**
**80639 München (DE)**

(54) **Method for the determination of biological age in human beings**

(57)    The present invention relates to methods for the determination of the biological age of a human being. This method is based on the findings of a multinational study screening for biomarkers of aging in humans.

Figure 1

**Description**

The present invention relates to methods for the determination of the biological age of a human being. This method is based on the findings of a multinational study screening for biomarkers of aging in humans.

Aging has been defined as the time-dependent decline of functional capacity and stress resistance, associated with increased risk of morbidity and mortality. Aging is a process that affects most if not all tissues and organs of the body. Moreover, cross-talk can occur between multiple physiological systems, e.g. cognitive or metabolic systems, which may influence the aging of the immune system. The mechanisms underlying the aging process are only beginning to be unraveled at the molecular level, yet there is clear evidence that the rate of aging differs significantly between members of the same animal species, including humans. In other words, the "biological age" may differ from the chronological age.

One important reason for such heterogeneity is individual genetic make-up. In several biological model systems, which are being extensively used in experimental gerontology, mutants have been isolated with either longer or shorter life span than the wild-type. In human beings, several rare hereditary, single-gene diseases are known to cause progeroid syndromes that are characterized by the early onset of some of the typical changes found in old individuals and premature death from diseases typical of old age. On the other hand, it is known that exceptional longevity in humans and the phenotype of "healthy aging" or "successful aging" have a hereditary, most likely polygenic component. This observation is the basis for the search for gene polymorphisms that are associated with this phenotype. The EC-funded "Genetics of Healthy Aging (GeHA)" study for example was a large-scale European effort focused on the systematic identification of the genetic components of human aging.

It is estimated, however, that genetic variation can account of maximally one third of the variability of the lifetime of human beings. Therefore other factors have to play a role. The influence of environmental factors, including nutrition, seems to be very important. This has been demonstrated experimentally in a wide variety of model systems, from *Saccharomyces cerevisiae* to rodents, and is very likely to hold true for humans as well.

But even under the most highly controlled experimental conditions, *i.e.* using model organisms and studying genetically identical individuals kept under identical environmental conditions, a significant degree of heterogeneity in lifetime can be observed, which has led to the conclusion that some element of chance (or some hitherto unknown and uncontrollable factor) will also play a role.

Although aging *per se* is a physiological phenomenon, it is also a very important risk factor for a wide variety of chronic and debilitating diseases. The rate of aging in humans is not uniform, due to genetic heterogeneity and the influence of environmental factors. Age-related changes in body function or composition that could serve as a measure of "biological" age and predict the onset of age-related diseases and/or residual lifetime are termed "biomarkers of aging". This term has been coined in analogy to biomarkers of specific chronic diseases, such as HIV infection, or biomarkers of exposure to toxins. Biomarkers of aging could be used to determine the rate of the aging process even in healthy human beings, and the identification of a relatively high biological age in a given person would indicate the necessity to initiate diagnostic procedures and early interventions to prevent or postpone the outbreak of overt age-related disease. Therefore, the availability of valid biomarkers of aging could represent an enormous step forward for Preventive Medicine.

The classical quantitative assessment of "aging" relies on the analysis of mortality curves of populations. In other words, individuals have to be followed up until the end of their lives in order to determine their "biological age" at any time point during their life. Therefore, at the level of a living individual, a reliable assessment of the state of aging, *i.e.* the state of the above-mentioned functional decline and a prediction of the risk of the onset of morbidity and the residual individual life expectancy are not possible with this method.

A strategy to solve this problem is the identification of age-related changes in body function or composition that could serve as a measure of "biological" age and predict the future onset of age-related diseases and/or residual lifetime more accurately than chronological age.

The American Federation for Aging Research has proposed the following criteria for a biomarker of aging. First, it must predict the rate of aging. In other words, it would tell exactly where a person is in their total life span. Further, it must be a better predictor of life span than chronological age. Second, it must monitor a basic process that underlies the aging process, not the effects of disease. Third, it must be able to be tested repeatedly without harming the person, for example by a blood test or an imaging technique. Fourth, it must be something that works in humans and in laboratory animals, such as mice. This is so that it can be tested in lab animals before being validated in humans. However, this last criterion is debatable because some biomarkers of human aging might exist that are of little relevance in model systems as some parameters/functions might be critical and/or limiting for the aging process only in complex organisms and not in simpler models. On the other hand, the primary identification and validation of biomarkers in model systems of aging can, of course, provide very promising candidates for targeted evaluation in humans.

Biomarkers of human aging are urgently needed for a variety of reasons. These include the identification of individuals at high risk of developing age-associated disease or disability. This would then prompt targeted follow-up examinations and, if available, prophylactic intervention or early-stage treatment of age-related disease. Furthermore, the availability of powerful biomarkers would allow the assessment of the efficacy of forthcoming pharmacological and other interventions (including optimization of micronutrient intake and other dietary components or physical activity) currently being developed and aimed to lower the risk of age-associated disease even in individuals without accelerated aging.

In view of the rapidly increasing average life expectancy of human beings world-wide, the prevalence of age-related diseases is likely to increase as well. This necessitates effective new strategies for prevention and early diagnosis of such conditions.

In order to determine biological age, previous studies have either relied on conventional clinical chemistry parameters measured in blood or its components or other body fluids as potential biomarkers of human aging, such as e.g. markers of kidney function, or on individual molecular parameters measured in blood or its components, such as e.g. telomere length. Occasionally, such measurements also comprised small groups of parameters and some studies also comprised physiological parameters, such as e.g. lung function, although these require full cooperation and motivation of the person to be tested.

However, conventional clinical chemistry parameters on their own do not reflect the specific changes occurring during aging at the molecular level. Therefore, analyses exclusively based on such parameters are not very powerful. Further, many individual candidate biomarkers of aging have been proposed, but in all cases their variability in cross-sectional studies is considerable, and therefore no single measurement has so far proven to yield a useful biomarker of aging on its own, probably due to the multi-causal and multi-systemic nature of aging. In addition, any assessment of biological age that requires full cooperation and motivation of the person to be tested is prone to errors and labor-intensive, which restricts their practical use at a wider scale.

Accordingly, the technical problem underlying the present invention is to provide a method for the determination of the biological age of a human being using a valid set of biomarkers of aging in humans that is based on biochemical and molecular analyses of blood and its components and/or urine and/or cellular samples. This set of biomarkers should be applicable to humans in the middle age range (e.g. 30 to 80 years) and should serve as a valuable diagnostic tool for preventive medicine by enabling identification of healthy persons whose aging process is accelerated and who thus are likely to be affected by typical age-related diseases at relatively young chronological age.

The solution to the above technical problem is achieved by the embodiments characterized in the claims.

In particular, in a first aspect, the present invention relates to a method for the determination of the biological age of a human being, comprising the steps of:

(a) providing one or more biological samples, selected from the group consisting of whole blood, serum, plasma, and urine, from the human being,

(b1) in case the human being is female, determining at least the following biomarkers (i) to (x) in the respective biological samples, wherein the necessary biological samples are provided in step (a):

(i) cumulative level of cytosine methylation at gene positions ELOVL_2_CpG_11, ELOVL_2_CpG_12, ELOVL_2_CpG_13, and ELOVL_2_CpG_14 in blood cells;

(ii) cumulative level of cytosine methylation at gene positions ELOVL_2_CpG_15, ELOVL_2_CpG_16, and ELOVL_2_CpG_17 in blood cells;

(iii) cumulative level of cytosine methylation at gene positions FHL_2_CpG_11, and FHL_2_CpG_12 in blood cells;

(iv) cumulative level of cytosine methylation at gene positions FHL_2_CpG_13, FHL_2_CpG_14, and FHL_2_CpG_15 in blood cells;

(v) cumulative level of cytosine methylation at gene positions FHL_2_CpG_16 and FHL_2_CpG_17 in blood cells;

(vi) concentration of dehydroepiandrosterone sulfate in plasma;

(vii) concentration of ferritin in serum;

(viii) decadic logarithm of the ratio between the concentration of NGA2F glycan in serum and the concentration of NA2F glycan in serum;

(ix) concentration of alpha-tocopherol in plasma;

(x) concentration of cysteine in plasma; or

(b2) in case the human being is male, determining at least the following biomarkers (i) to (x) in the respective biological samples, wherein the necessary biological samples are provided in step (a):

(i) cumulative level of cytosine methylation at gene positions ELOVL_2_CpG_11, ELOVL_2_CpG_12, ELOVL_2_CpG_13, and ELOVL_2_CpG_14 in blood cells;

(ii) cumulative level of cytosine methylation at gene positions ELOVL_2_CpG_15, ELOVL_2_CpG_16, and ELOVL_2_CpG_17 in blood cells;

(iii) cumulative level of cytosine methylation at gene positions FHL_2_CpG_13, FHL_2_CpG_14, and FHL_2_CpG_15 in blood cells;

(iv) level of cytosine methylation at gene position FHL_2_CpG_18;

(v) concentration of creatinine in urine;

(vi) ratio of the number of CD3$^+$CD4$^+$ cells in whole blood to the number of CD8$^+$ cells in whole blood;

(vii) concentration of dehydroepiandrosterone sulfate in plasma;

(viii) concentration of alpha-2-macroglubulin in serum;

(ix) concentration of lycopene in plasma;

(x) concentration of cysteine in plasma; and

(c) determining the biological age of the human being based on the biomarkers determined in step (b1)/(b2).

In the context of the present invention, the terms "chronological age" and "biological age" or "bioage" are used and discriminated from each other. The term "chronological age" relates to the age of a human being as calculated as the amount of time since the person's day of birth. By way of example, a person that is born on April 27, 1975 has on February 22, 2013 a chronological age of 37 years, 9 months, and 26 days, or 37.816 years. The term "biological age" relates to a human being's bodily age, *i.e.* an artificial value that denotes how far the body has aged in terms of age-related changes in body function or composition. Again by way of example, the above person having a chronological age of 37.816 years might have (i) a lower biological age, e.g. 30 years, indicating that said person's body shows less age-related changes in body function or composition as compared to the average population, (ii) a higher biological age, e.g. 45 years, indicating that that said person's body shows more age-related changes in body function or composition as compared to the average population, or (iii) a biological age that is more or less identical to the person's chronological age, indicating that said person's body shows age-related changes in body function or composition that correspond largely to those of the average population.

The biomarkers determined in the methods of the present invention, as well as methods for determining the biological age of a human being based on the determined biomarkers will be extensively discussed hereinafter. In this context, the small roman numerals assigned to the biomarkers herein and in the claims are always to be seen in the context of whether the biomarkers are determined for females or males, *i.e.*, by way of example, biomarkers (i) to (x) used for females differ from biomarkers (i) to (x) used for males; further, the same biomarker may have one numeral when used for females, and another when used for males. Sometimes hereinafter, biomarkers used for females or males are referred to as female or male biomarkers. Table 1 below provides the respective numerals for all biomarkers used in the present invention.

In a preferred embodiment of the method of the present invention, in case the human being is female, in step (b1) one or more of the following biomarkers (xi) to (xxviii) are determined in addition to the biomarkers (i) to (x), and the necessary biological samples are provided in step (a):

(xi) cumulative level of cytosine methylation at gene positions ELOVL_2_CpG_22, ELOVL_2_CpG_23, and ELOVL_2_CpG_24 in blood cells;

(xii) cumulative level of cytosine methylation at gene positions FHL_2_CpG_19, and FHL_2_CpG_20 in blood cells;

(xiii) concentration of NGA2FB glycan in serum;

(xiv) concentration of fibrinogen in plasma;

(xv) concentration of urea in plasma;

(xvi) concentration of glucose in serum;

(xvii) length of telomers in blood cells;

(xviii) concentration of triglycerides in serum;

(xix) concentration of immunoglobulin M (IgM) in serum;

(xx) concentration of adiponectin in serum;

(xxi) accumulated concentration of threonine and lactate in urine;

(xxii) concentration of homocysteine in plasma;

(xxiii) concentration of lycopene in plasma;

(xxiv) concentration of cholesterol in serum;

(xxv) concentration of glycosylated hemoglobin (HbA$_{1c}$) in whole blood;

(xxvi) concentration of glycine in urine;

(xxvii) number of T cell receptor excision circles (Trec) in 100,000 T cells from whole blood;

(xxviii) concentration of creatinine in urine.

In a further preferred embodiment of the method of the present invention, in case the human being is male, in step (b1) one or more of the following biomarkers (xi) to (xxiii) are determined in addition to the biomarkers (i) to (x), and the necessary biological samples are provided in step (a):

(xi) cumulative level of cytosine methylation at gene positions FHL_2_CpG_19, and FHL_2_CpG_20 in blood cells;
(xii) number of T cell receptor excision circles (Trec) in 100,000 T cells from whole blood;
(xiii) decadic logarithm of the ratio between the concentration of NGA2F glycan in serum and the concentration of NA2F glycan in serum;
(xiv) concentration of prostate specific antigen (PSA) in serum;
(xv) concentration of fibrinogen in plasma;
(xvi) concentration of albumin in plasma;
(xvii) concentration of glucose in serum;
(xviii) concentration of NGA2FB glycan in serum;
(xix) ratio of the concentration of Cu in plasma to the concentration of Zn in plasma;
(xx) ratio of the number of $CD16^+CD56^+$ cells in whole blood to the number of $CD45^+$ cells in whole blood;
(xxi) concentration of antibodies directed against *Clostridium tetani* in plasma;
(xxii) ratio of the number of $CD3^+CD8^+$ cells in whole blood to the number of $CD45^+$ cells in whole blood;
(xxiii) concentration of beta-hydroxyisovalerate in urine.

The present invention has been realized based on the findings of an ongoing multinational population study (sometimes referred to as "the study underlying the present invention" hereinafter) on approximately 3,300 subjects from eight European countries which aims to identify a set of biomarkers of aging which, as a combination of parameters with appropriate weighting, can measure biological age better than any marker in isolation. Two groups of subjects were be recruited, *i.e.* a large randomly recruited age-stratified group of individuals from the general population (labeled "RASIG"; ratio male : female = 1:1), covering the chronological age range of 35 to 74 years, and, for reasons of comparison and validation, a group of subjects born from a long-living parent and belonging to a family with long living sibling(s) already recruited in the framework of the EU Integrated Project GeHA (Genetics of Healthy Aging). For genetic reasons, such individuals ("GeHA offspring" or short "GO" hereinafter) are expected to age at a slower rate.

Approximately 300 different clinical chemistry, genetic, cellular or molecular biomarkers were tested, including classical ones for which a correlation with age has been known, new ones that are based on recent findings, and parameters that are based on recent research on mechanistic aspects of aging, conducted by study participants. All analyses have been done on coded samples. Analyses resulted in a large amount of data containing the values for the determined biomarkers for all study subjects (sometimes referred to as "the data provided in the study underlying the present invention" hereinafter).

Bioinformatics was used in order to extract a robust set of biomarkers of human aging from the large amounts of data obtained. This was done by using a graphical workbench for the entire analysis process, including data access in databases, data transformation, initial investigation, powerful predictive analytics, visualization and reporting. Further, the "leaps" package which is a component of the free statistics software "R" was used for statistical analysis and calculations. This package can perform an exhaustive search for the best subsets of the variables in x for predicting y in linear regression, using an efficient branch-and-bound algorithm. Furthermore, the package "stats" which is also a component of "R", was used for the fitting of linear models. This package can be used to carry out regression, single stratum analysis of variance and analysis of covariance.

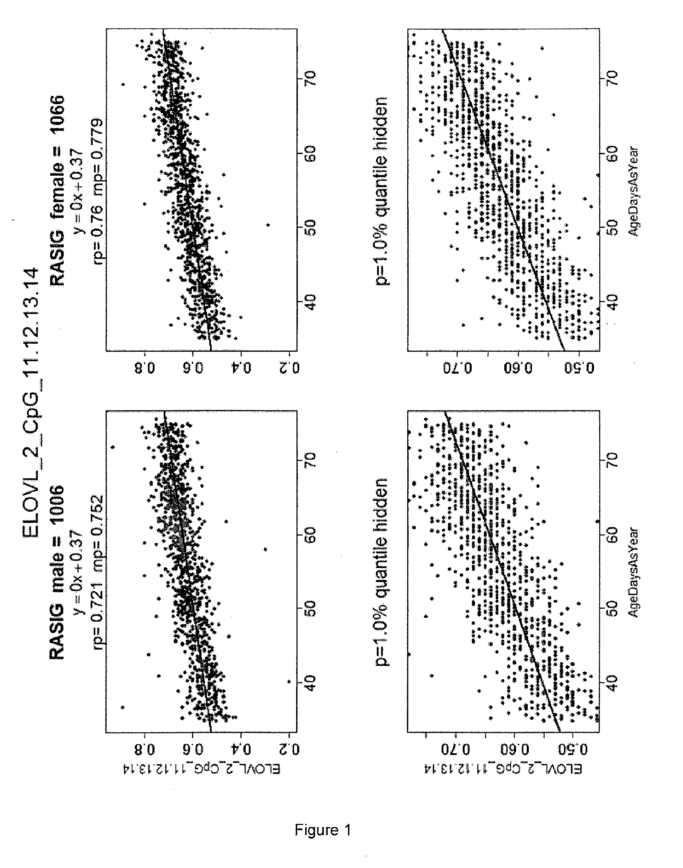

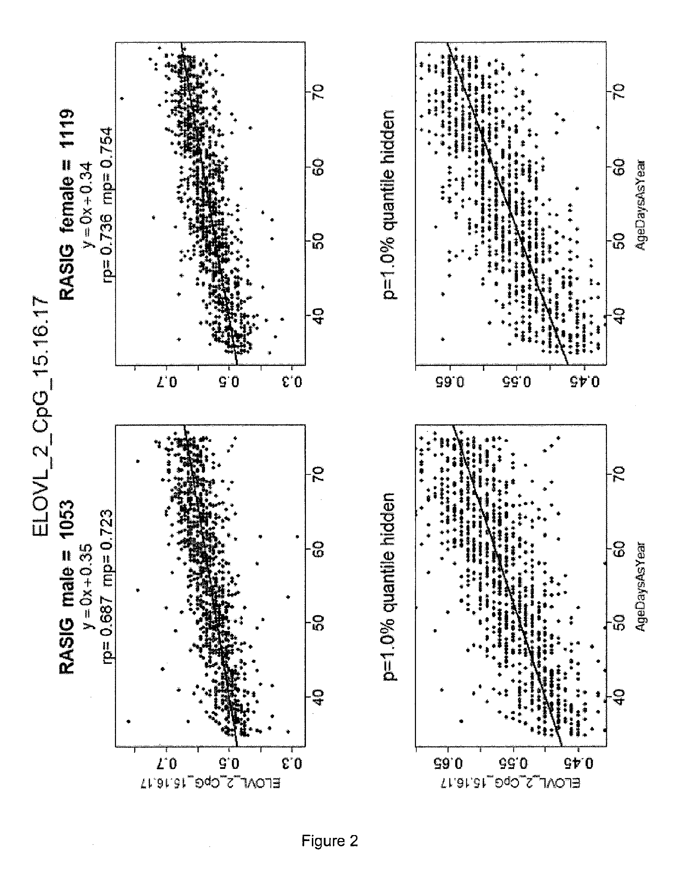

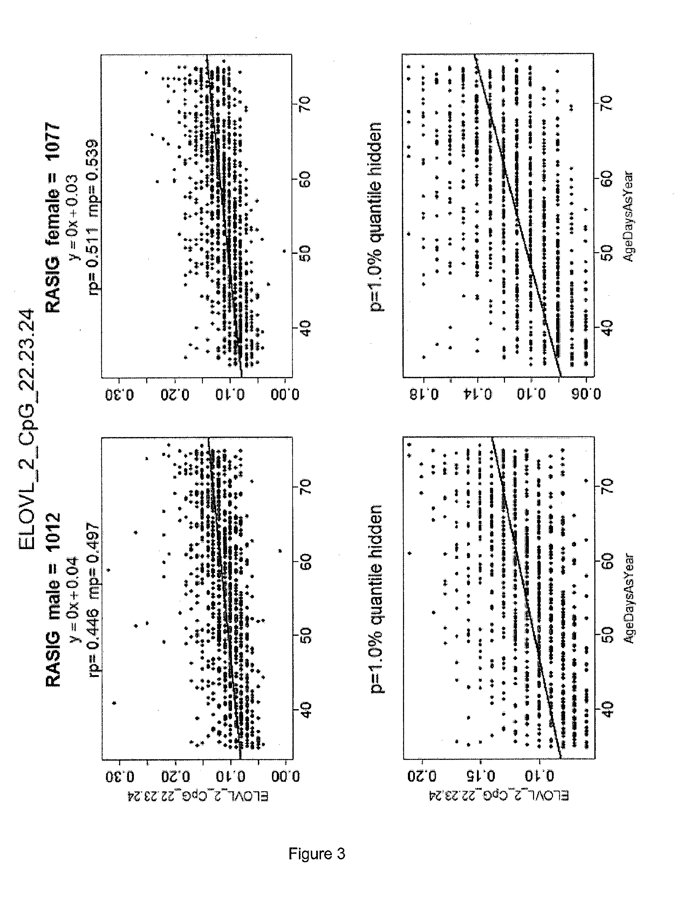

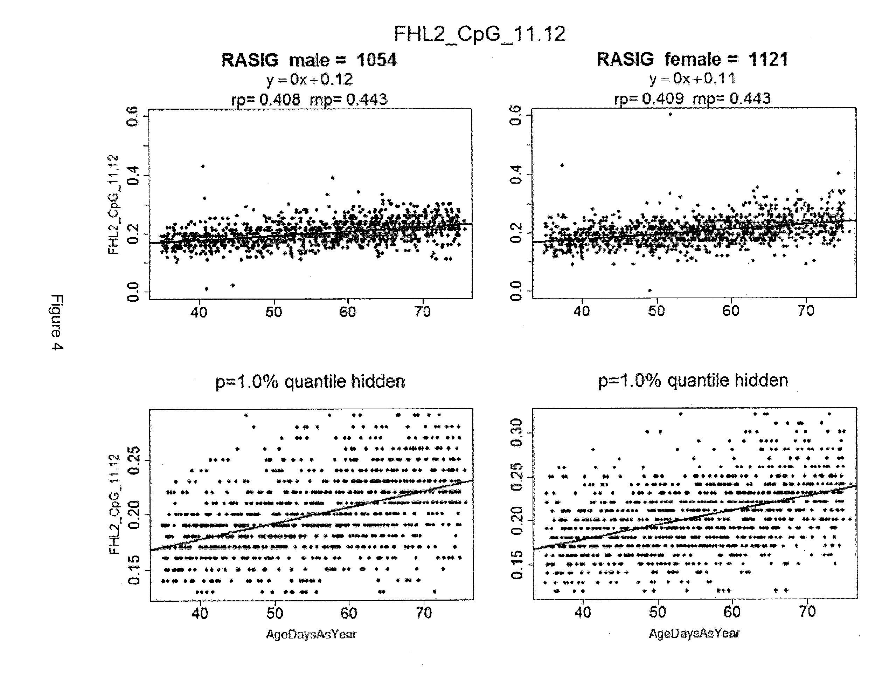

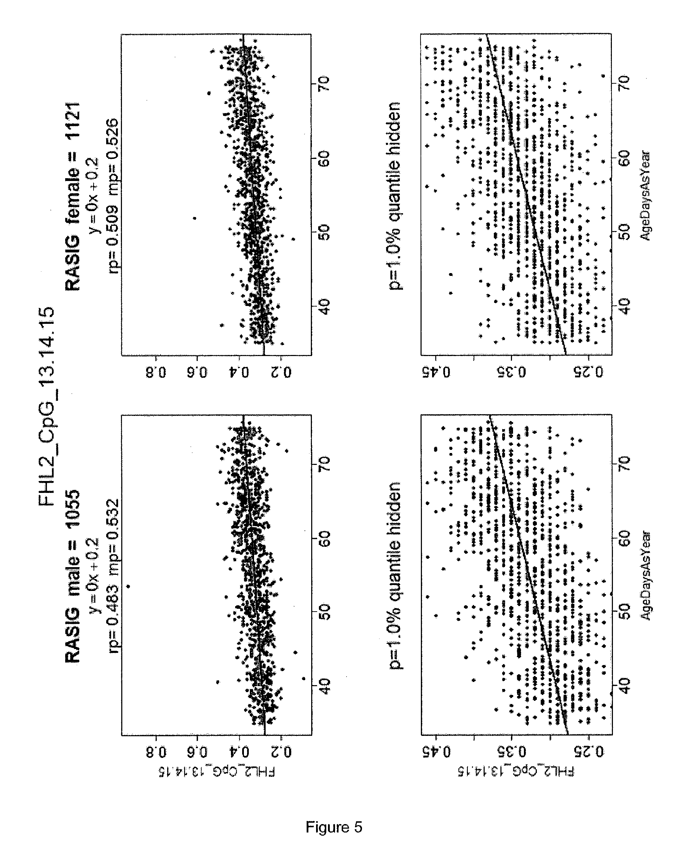

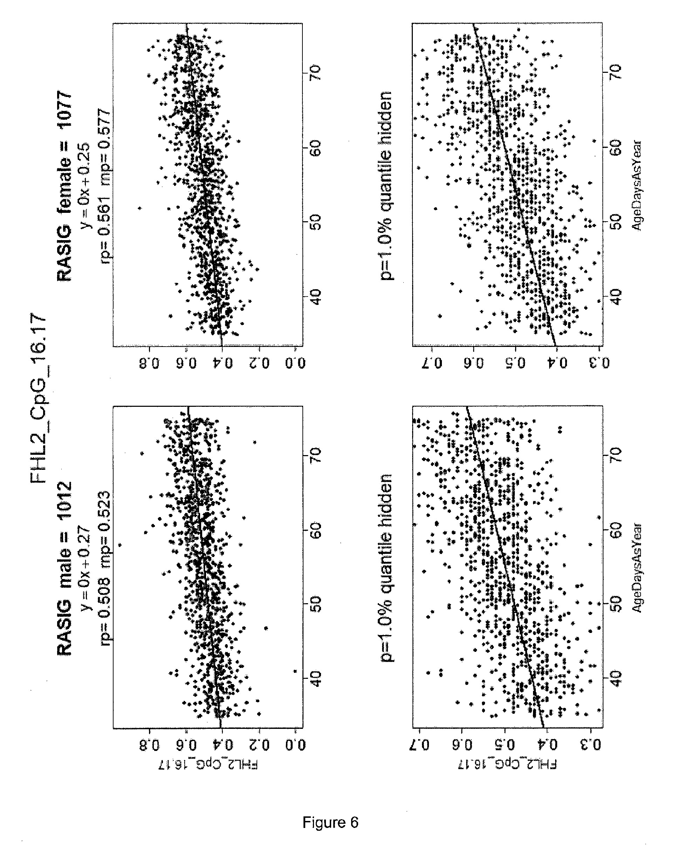

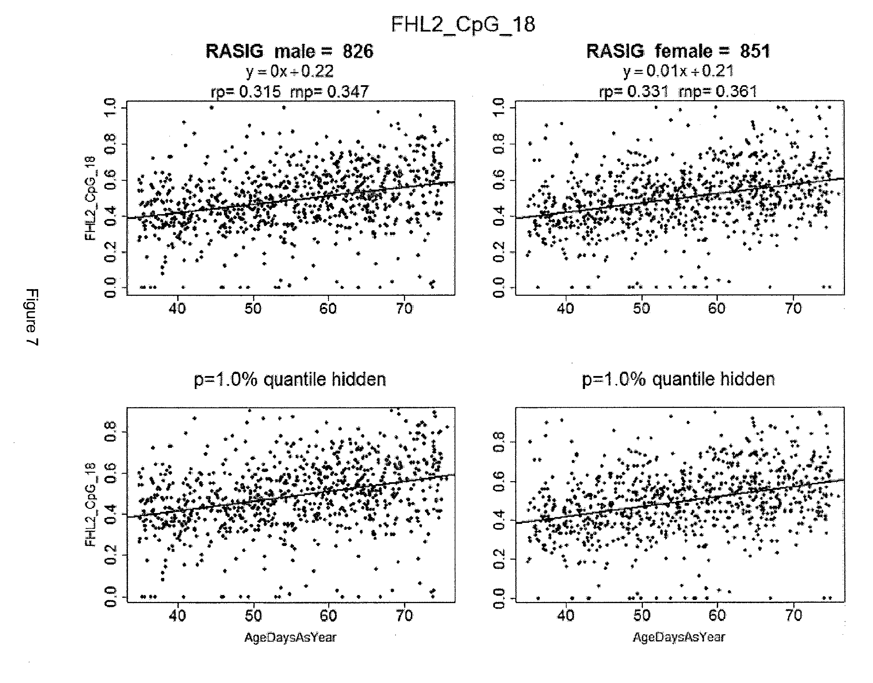

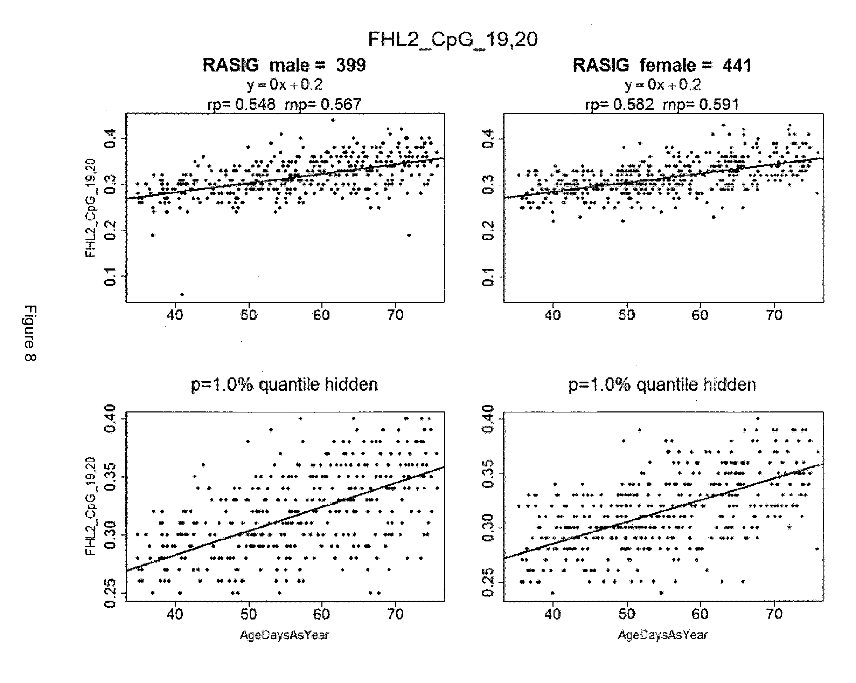

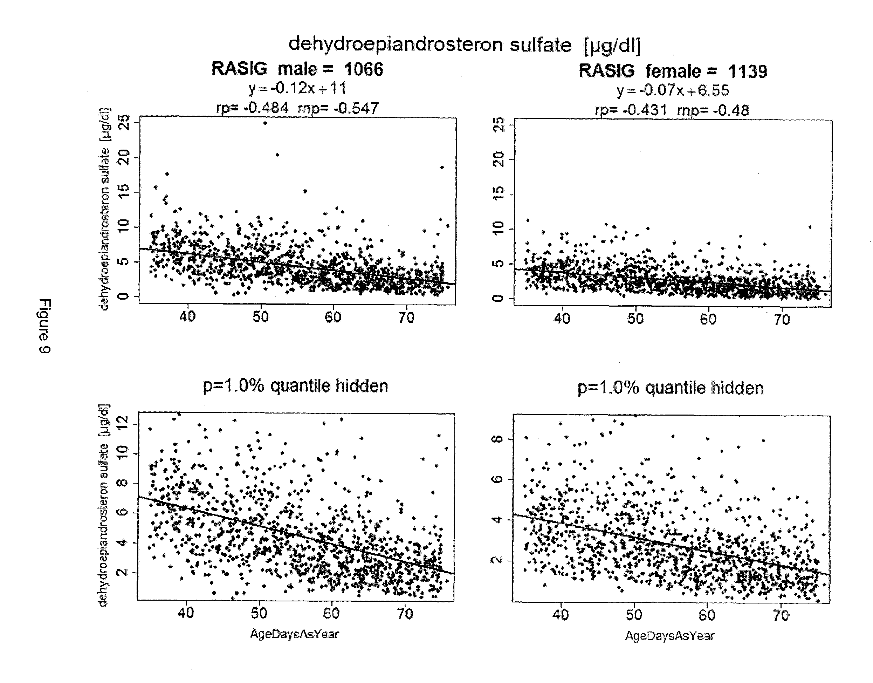

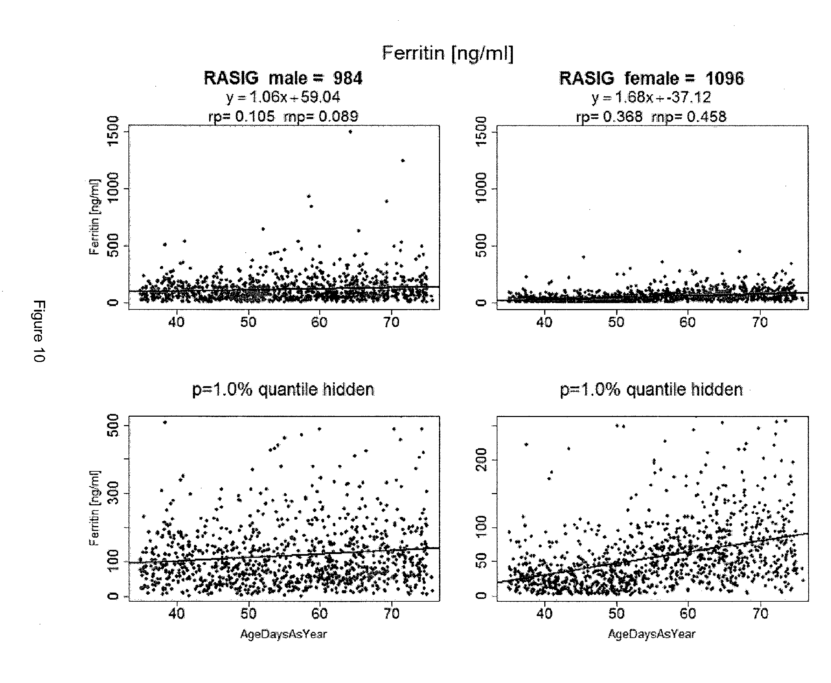

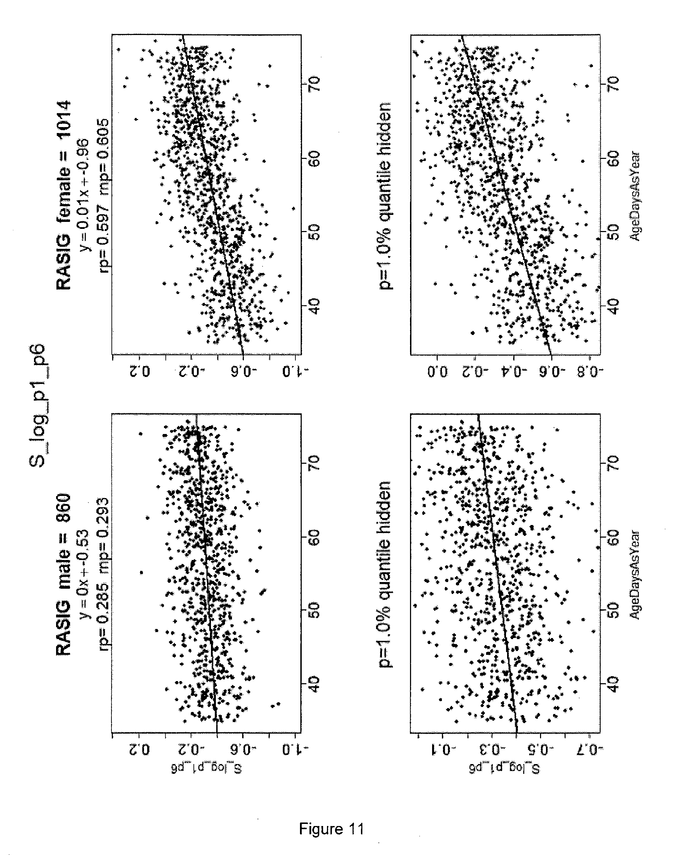

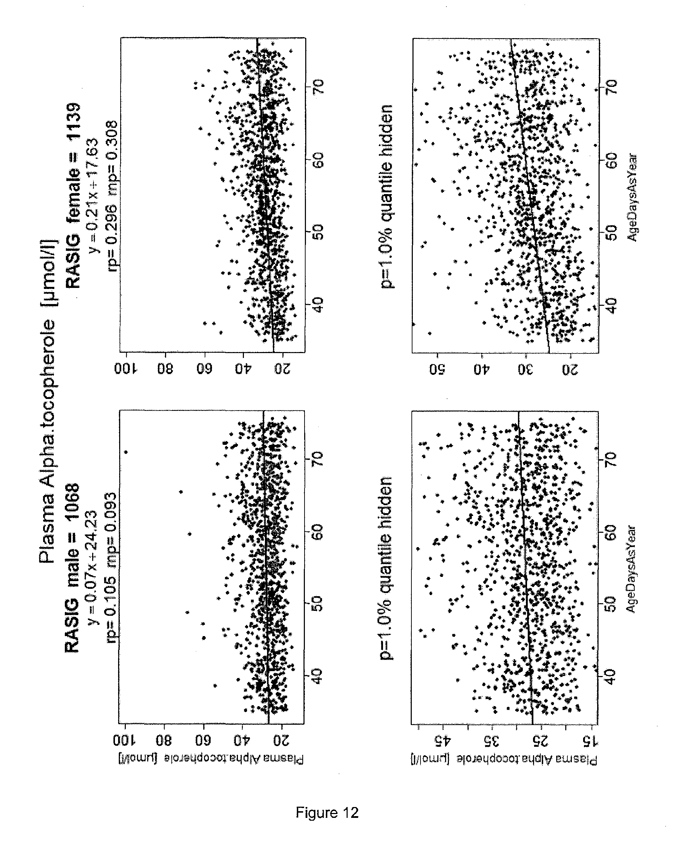

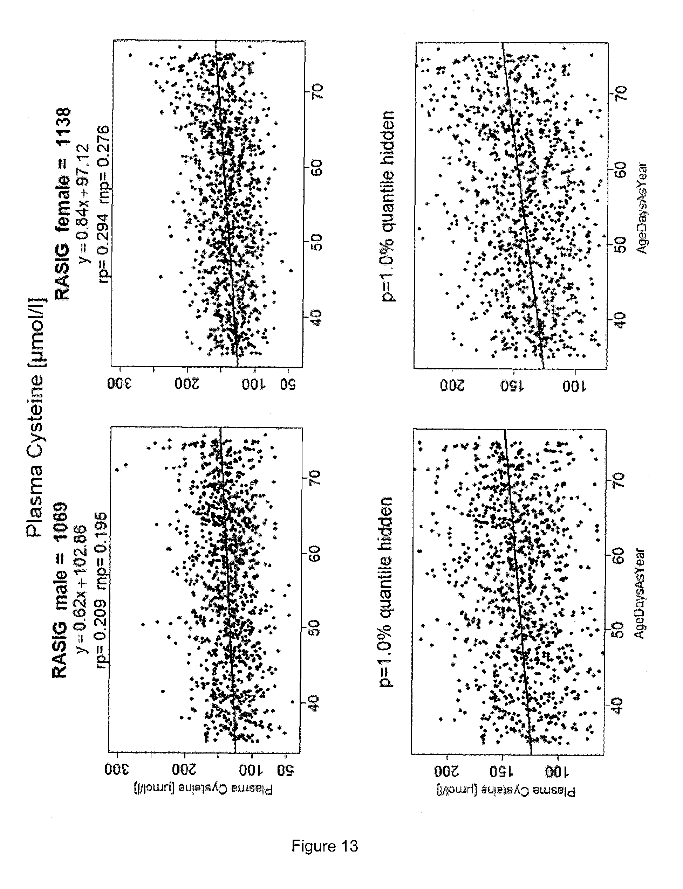

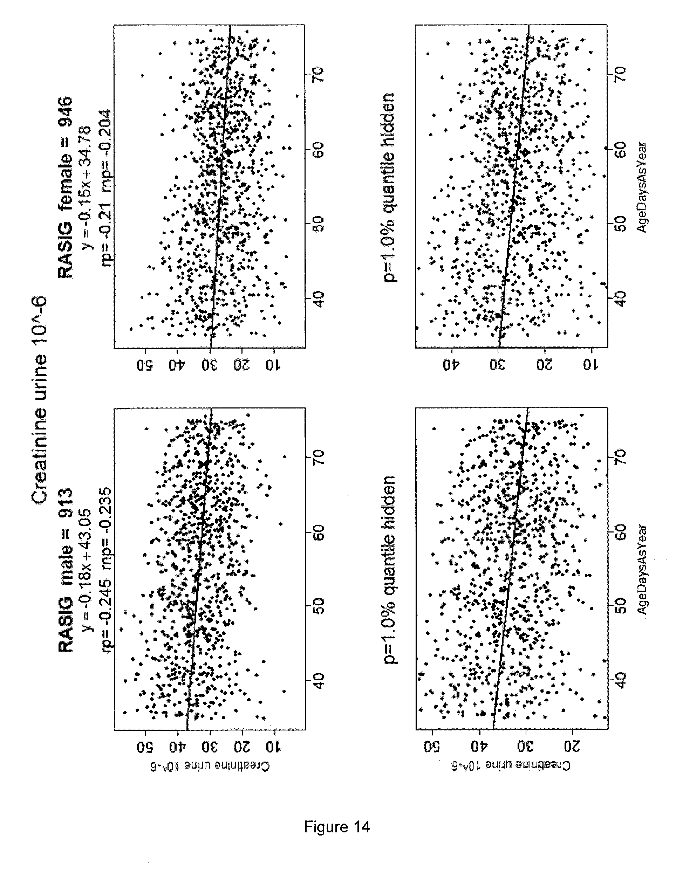

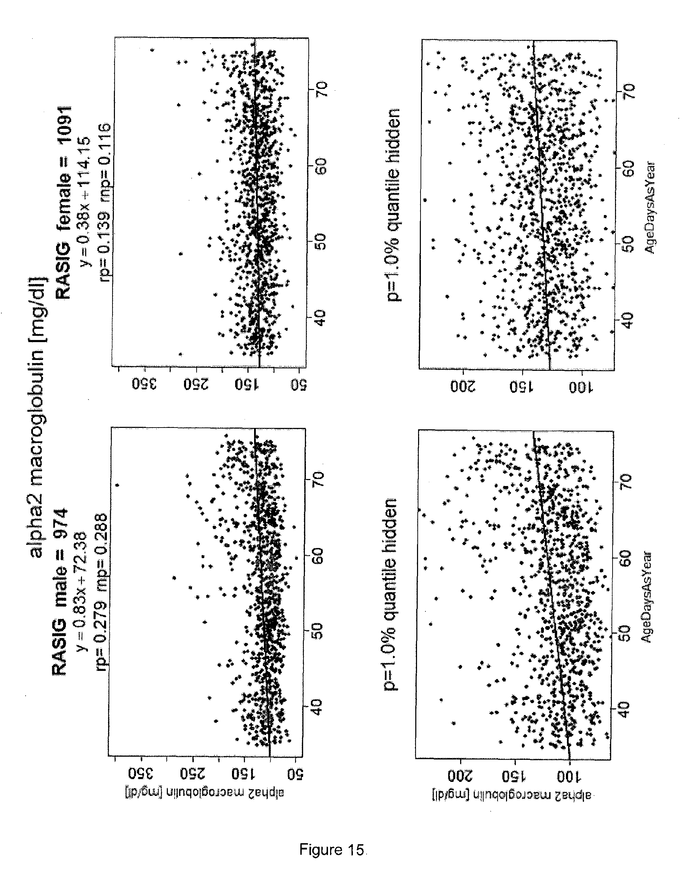

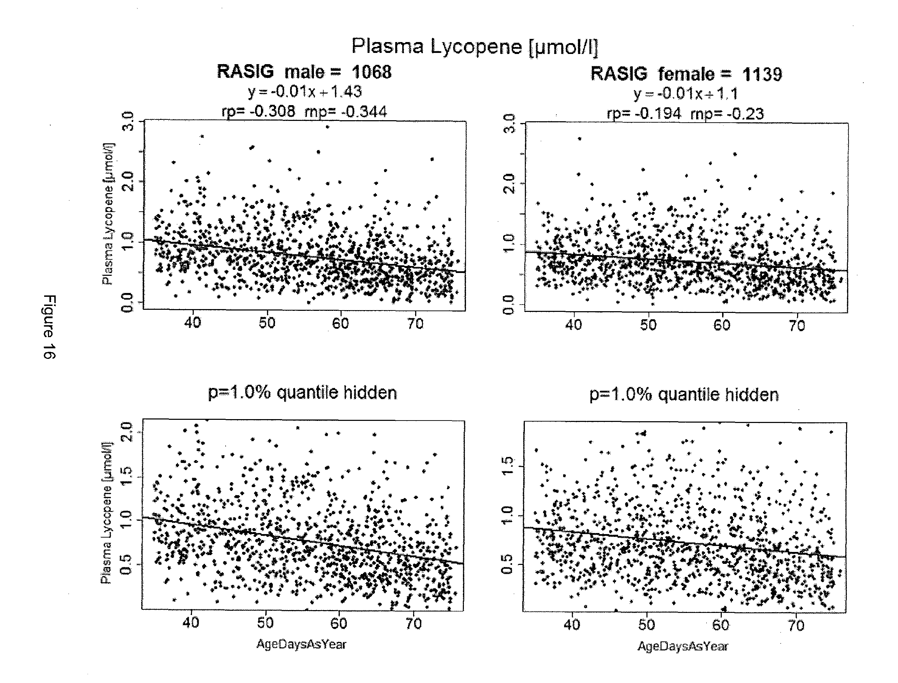

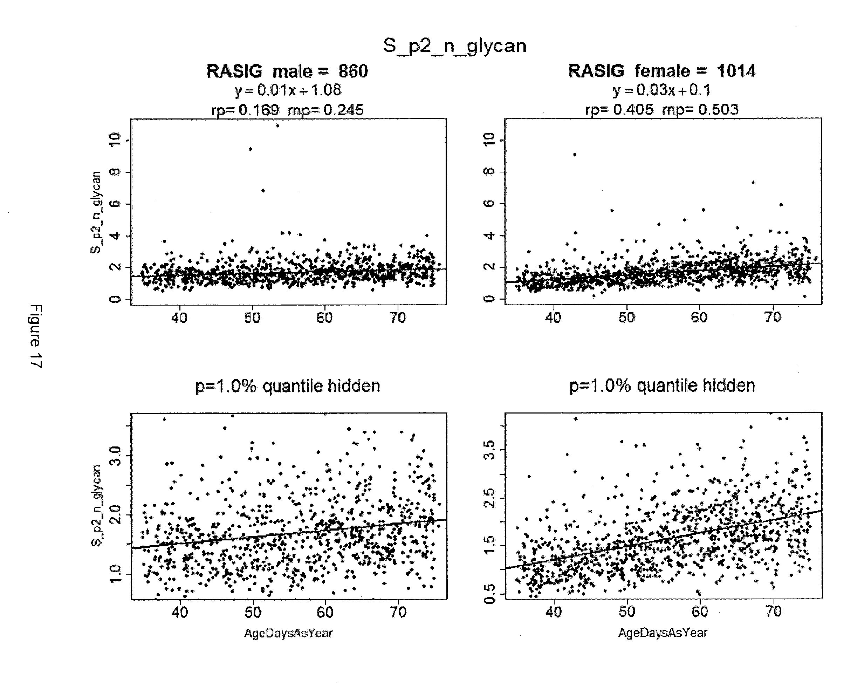

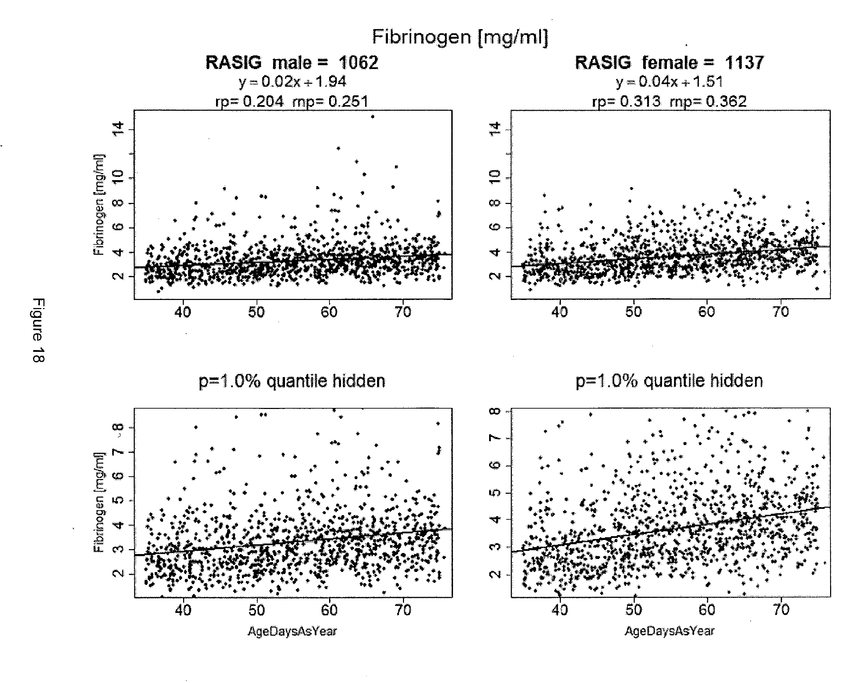

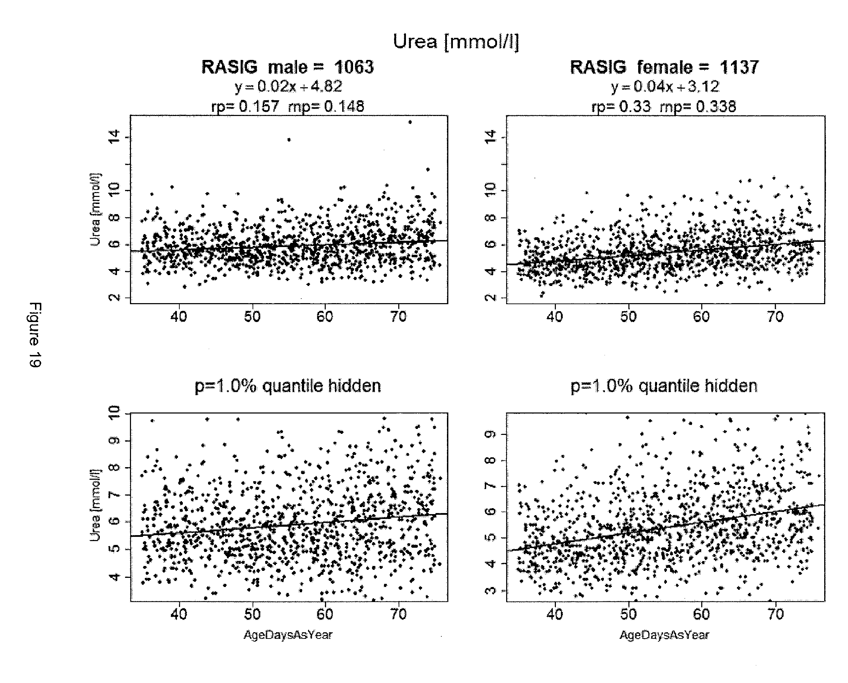

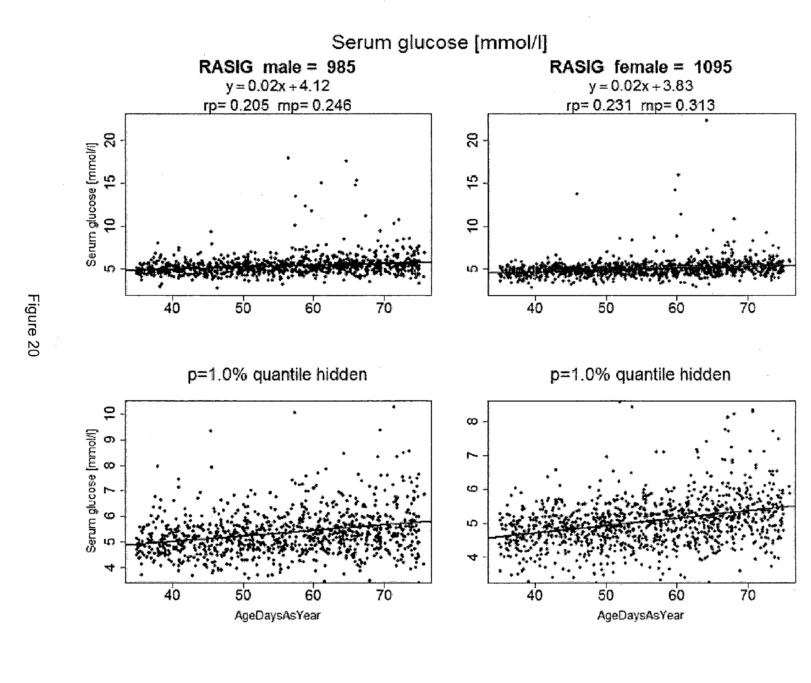

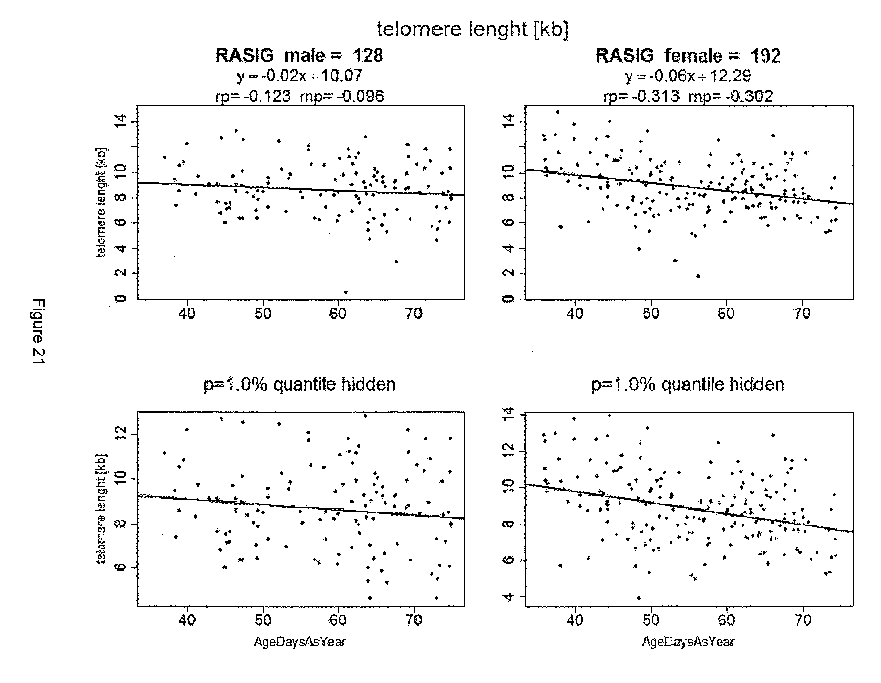

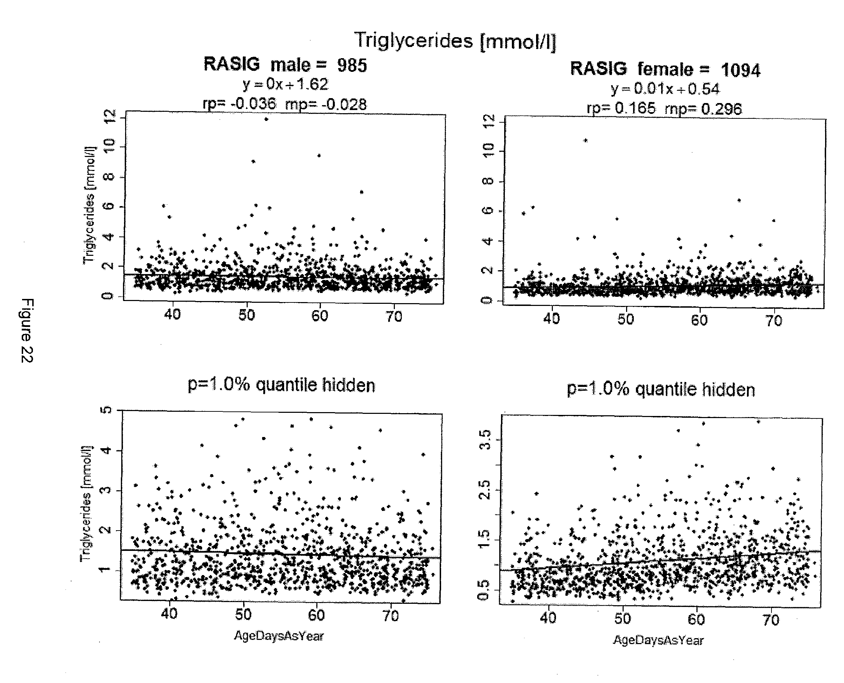

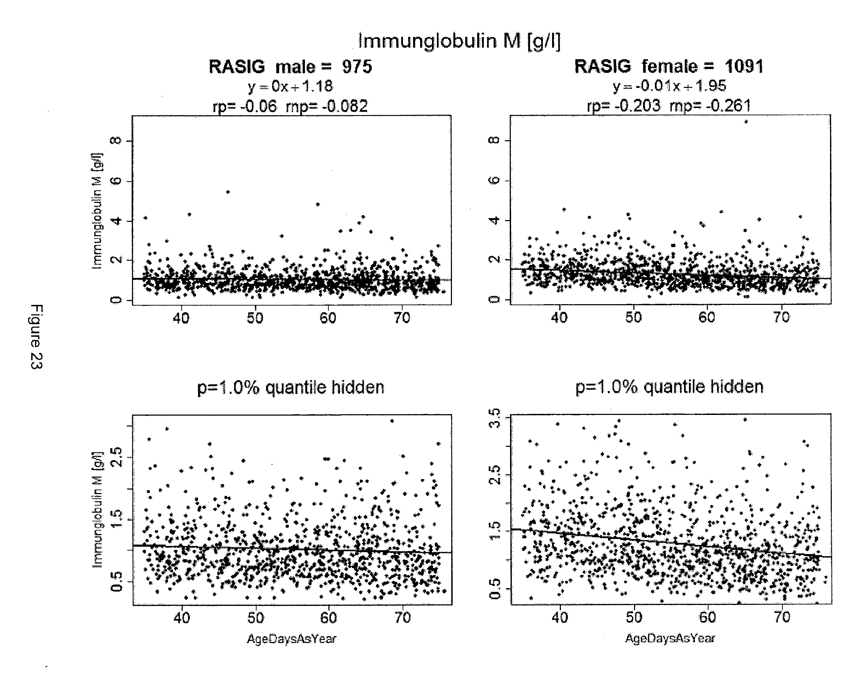

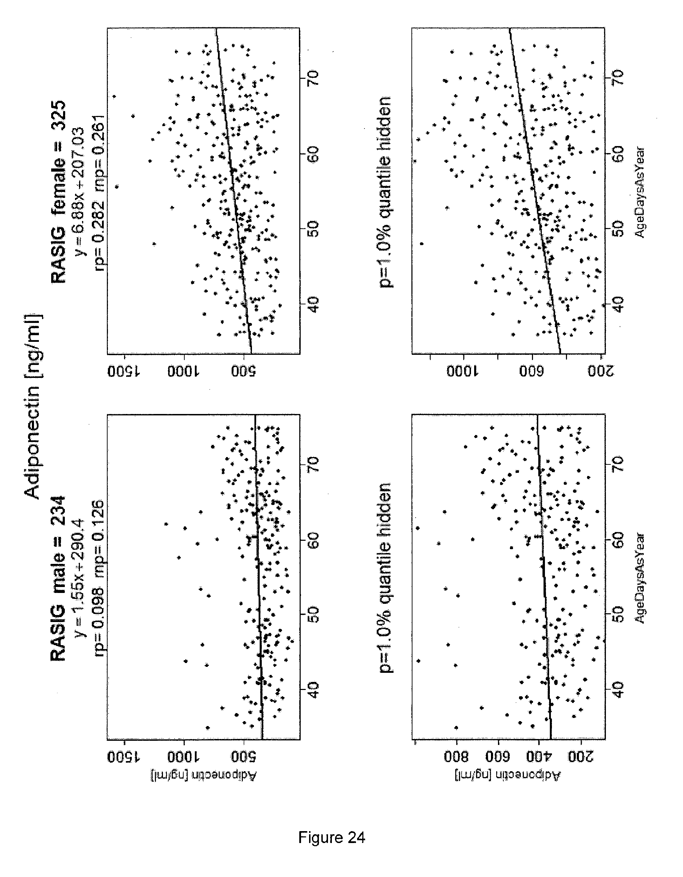

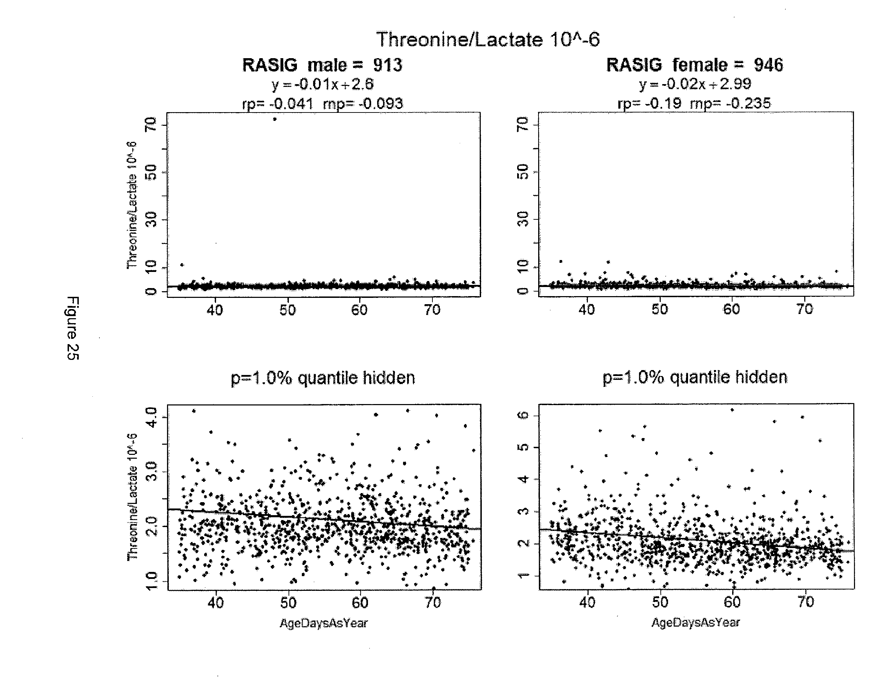

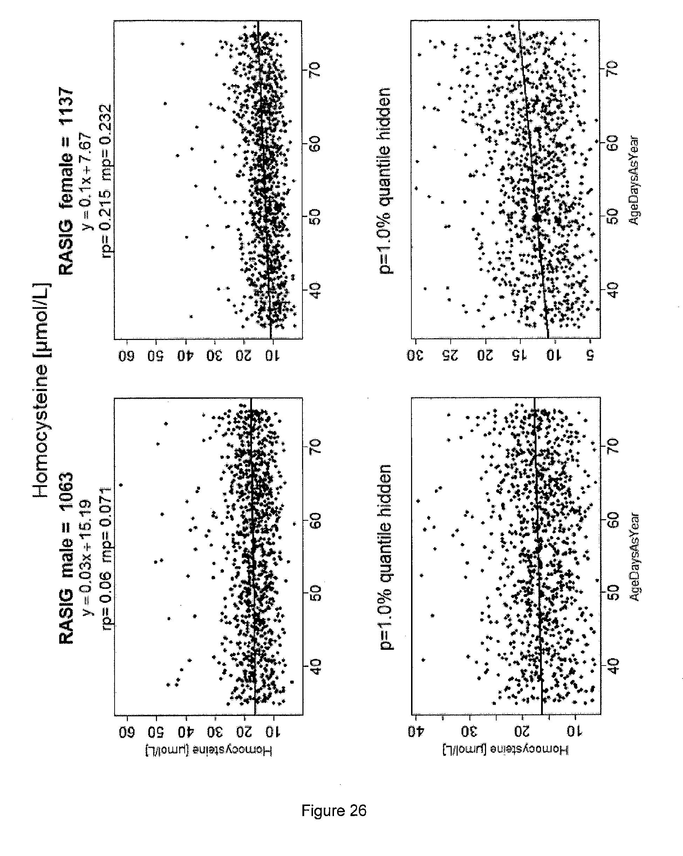

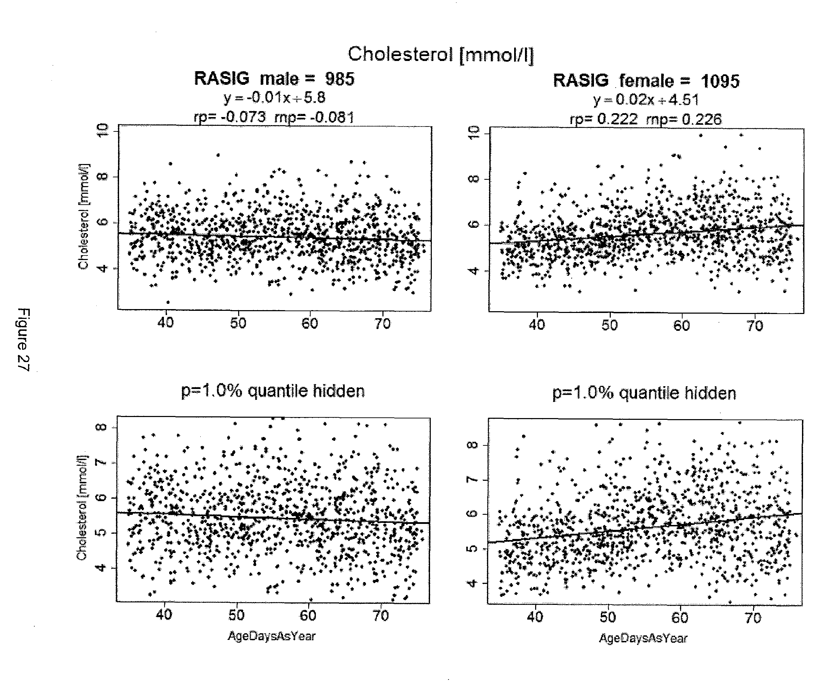

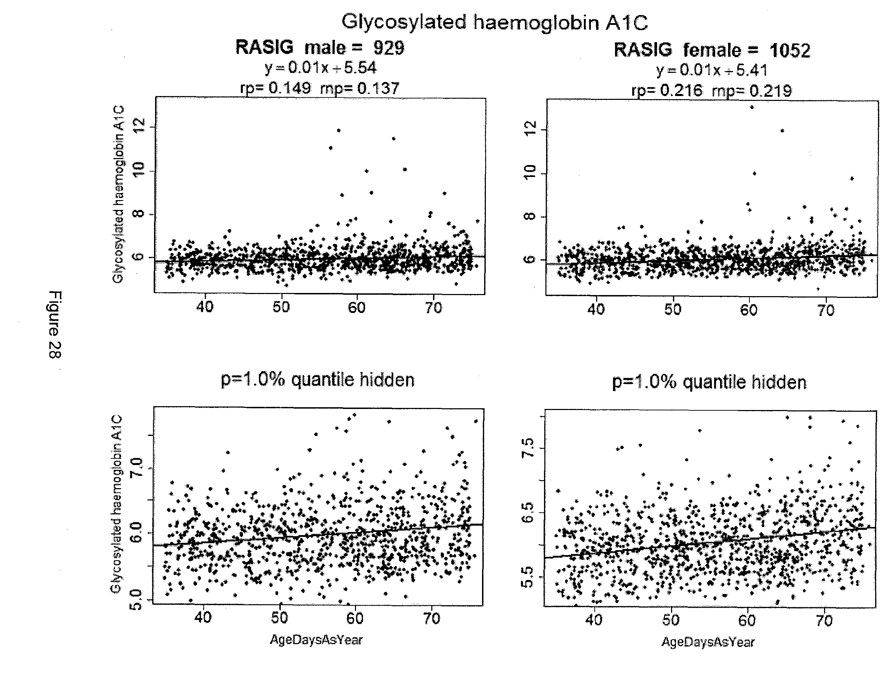

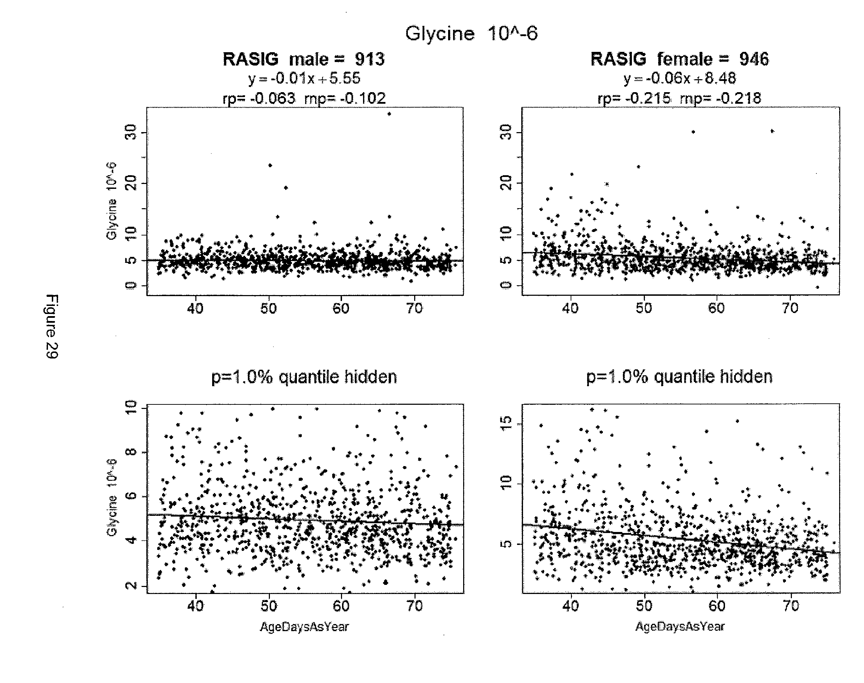

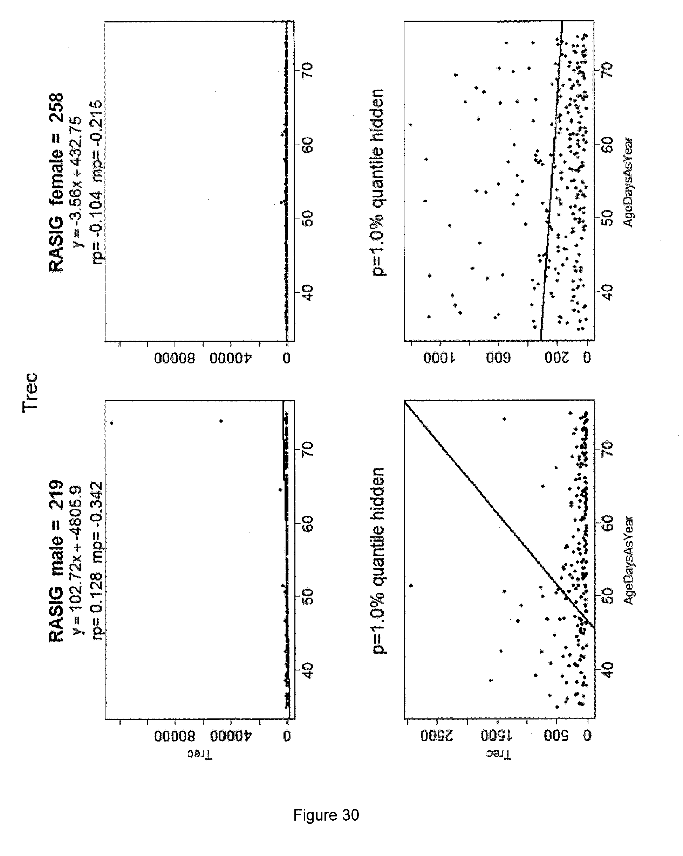

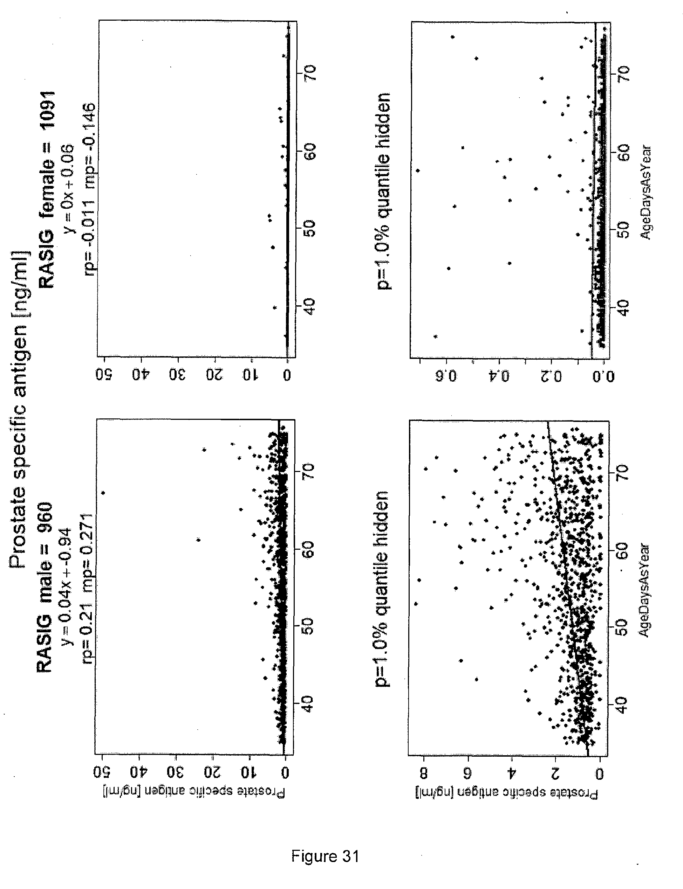

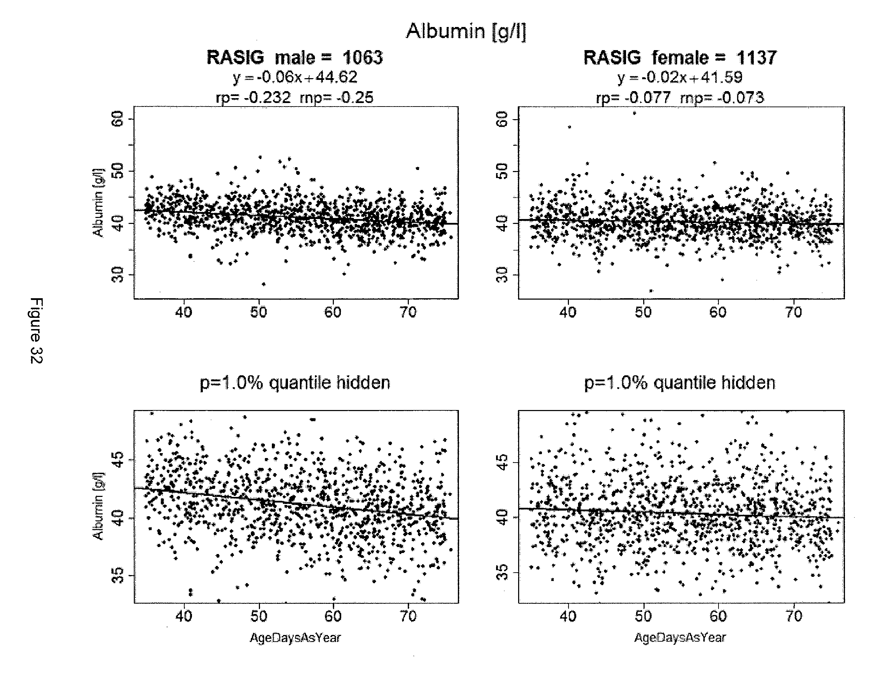

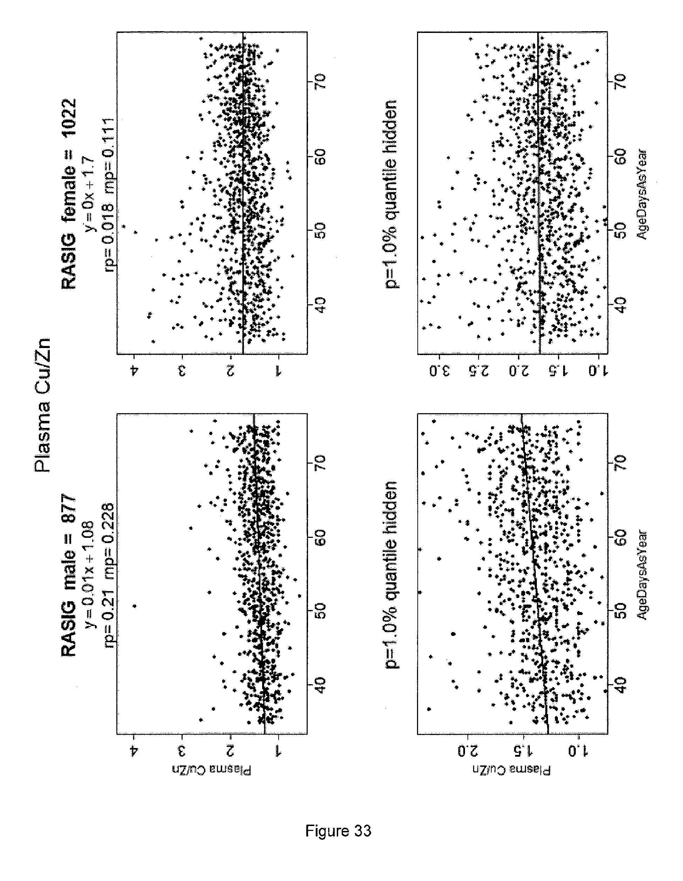

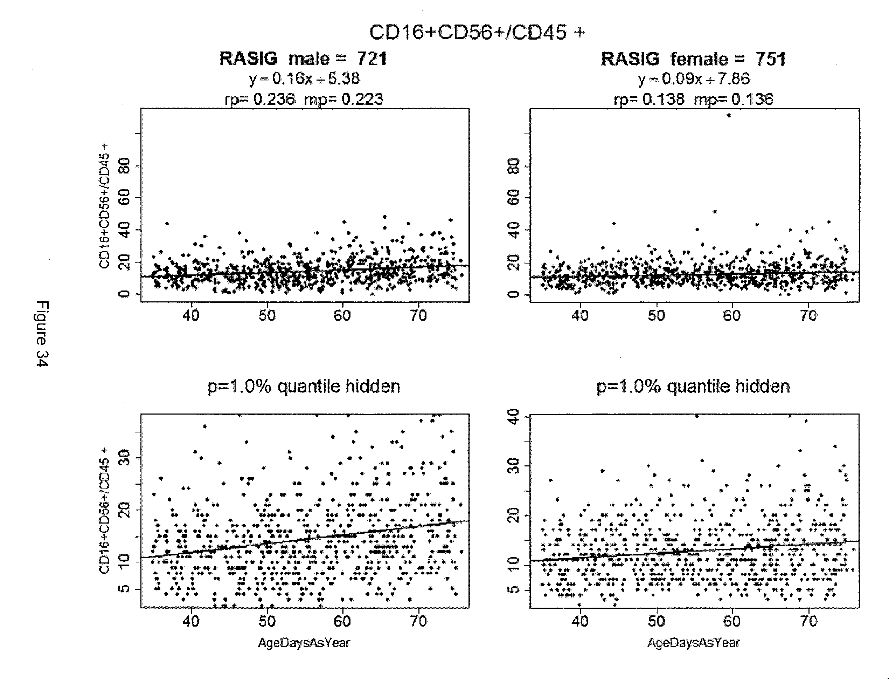

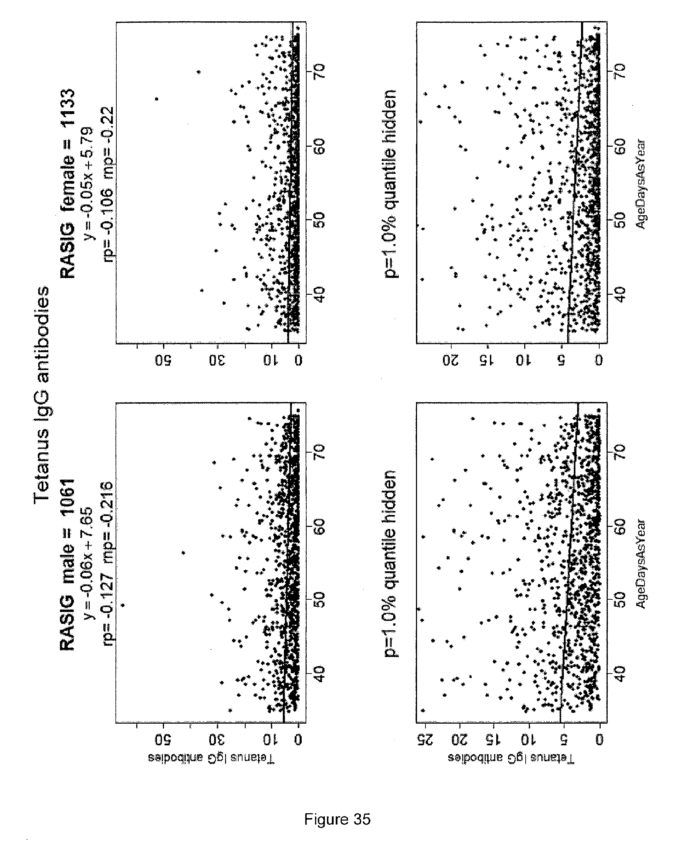

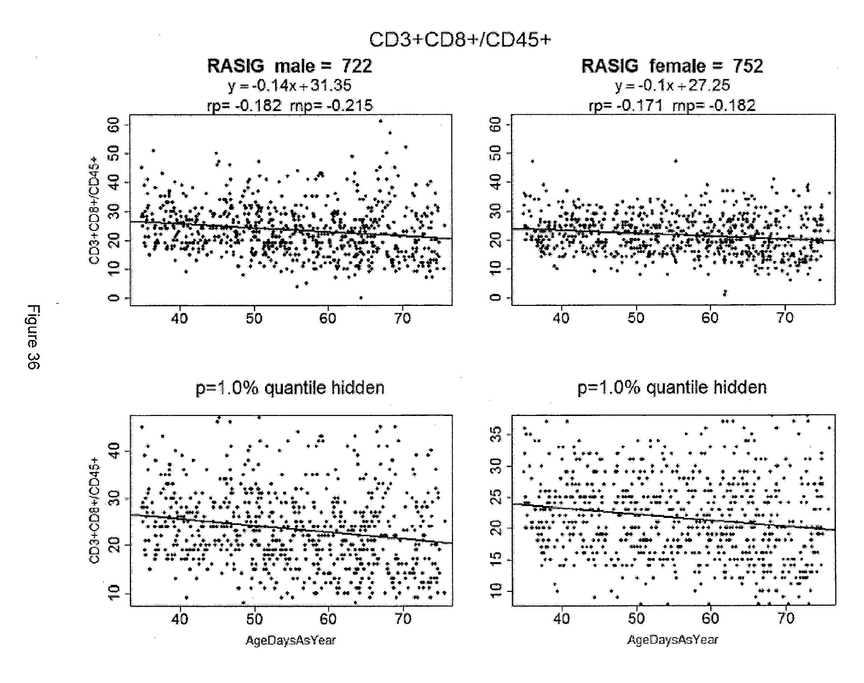

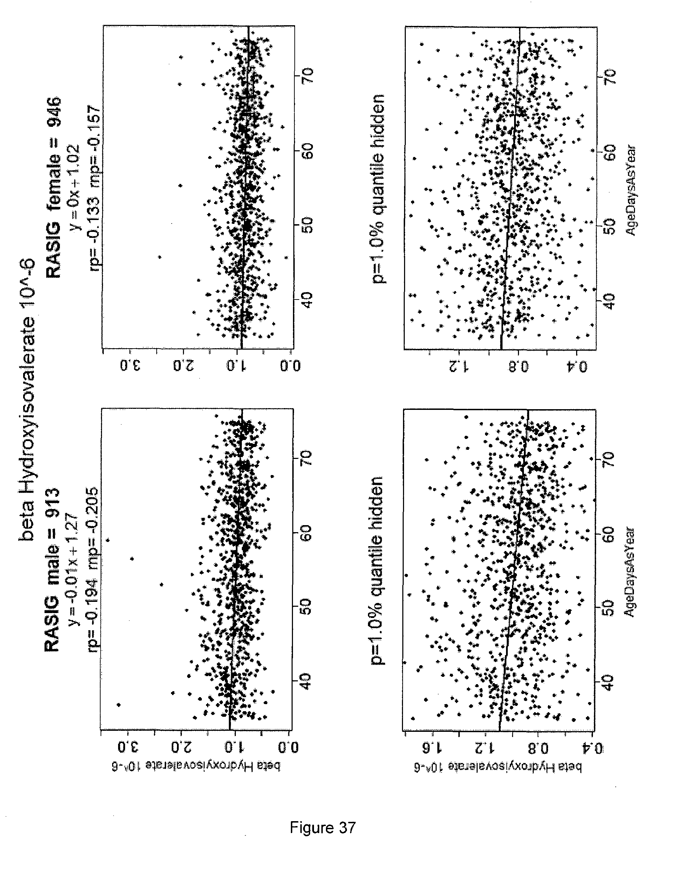

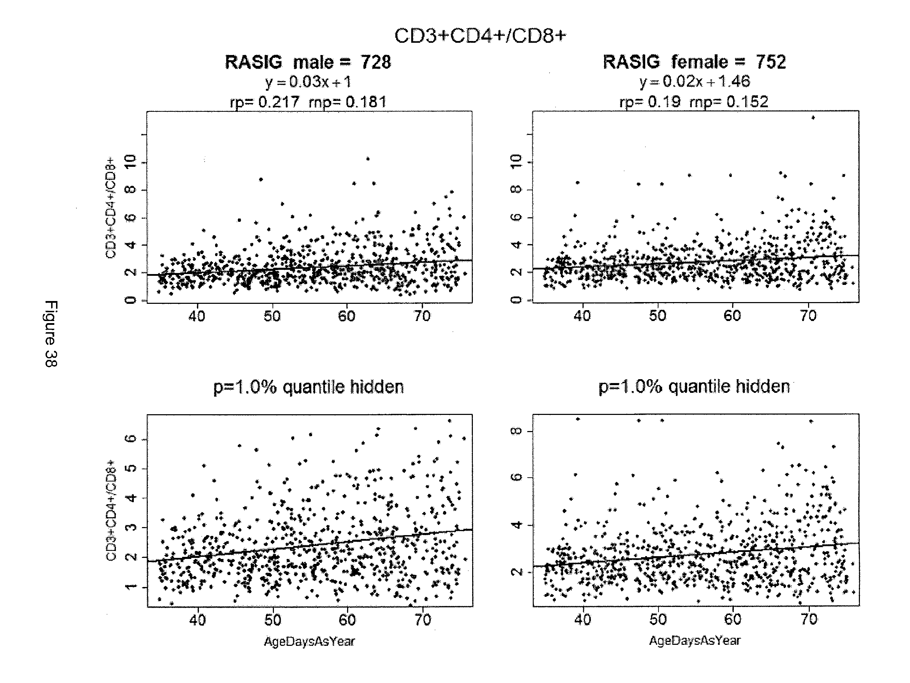

More specifically, in the study underlying the present invention, a correlation between each determined biomarker and age has been determined, resulting in (i) a parametric coefficient of correlation (rp), (ii) a non-parametric coefficient of correlation (rnp), (iii) the slope of the linear regression curve (a), and (iv) the intercept of the linear regression curve (c). This was done separately for female and male subjects, since sex differences in the quality of biomarkers were expected. The non-parametric coefficient of correlation (rnp) for the biomarkers used in the present invention is shown in Table 2, below, and the respective graphs are shown in Figures 1 to 38. Then, the 30 biomarkers showing the best correlation with age were chosen, independent of whether the correlation was positive or negative. Subsequently, a regression subset analysis for the chosen parameters was performed, resulting in a subset of the ten best biomarkers as far as the capacity to predict the chronological age of the subjects was concerned. Then, for these ten parameters, a multiple linear regression model was build, resulting in a weighting factor for each biomarker (weighting factors $b_1$ to $b_{10}$), a correlation coefficient $r^2$ for the multiple linear regression, and a intercept c for the multiple linear regression. Based on these data, the biological age of any individual subject can be calculated according to the formula

$$B = \sum_{i=1}^{10} b_i x_i + c$$

wherein B is the apparent biological age and $X_1$ to $X_{10}$ are the determined values of the respective biomarkers. In order to alleviate the usage of the biological age values, a strictly monotone transformation is applied to the apparent biological age which ensures that the average biological age approximately equals the chronological age in randomly selected even-aged groups of individuals. The transformation is given by the formula

$$Bioage = \frac{B - mean(a)}{r^2} + mean(a)$$

wherein Bioage is the actual, corrected biological age and mean(a) is the mean age of the whole test population.

However, according to the embodiments described above, one or more additional biomarkers can be used for the calculation of the biological age of a human being, *i.e.* for females one or more of the above biomarkers (xi) to (xvxiii), and for males one or more of the above biomarkers (xi) to (xxiii). In case any of these additional biomarkers are used, as well as in case only the biomarkers (i) to (x) are used, in step (c) determining the biological age of the human being based on the biomarkers determined in step (b1)/(b2) comprises the steps:

(c1) establishing a multiple linear regression model for the determined biomarkers based on the data provided in the study underlying the present invention;
(c2) determining from the multiple linear regression model established in step (c1)

(i) the correlation coefficient $r^2$,
(ii) the weighting factors $b_i$ for each determined biomarker, wherein i is 1 through n, and n is the number of biomarkers determined, and
(iii) the intercept c;

(c3) calculating an apparent biological age B according to formula (1)

$$B = \sum_{i=1}^{n} b_i x_i + c \qquad (1)$$

wherein

$b_i$    are the weighting factors determined in step (c2),
$x_i$    are the values of the respective biomarkers as determined in step (b1)/(b2), wherein i is 1 to n and
c    is the intercept determined in step (c2); and

(c4) calculating the corrected biological age (Bioage) according to formula (2)

$$Bioage = \frac{B - mean(a)}{r^2} + mean(a) \qquad (2)$$

wherein

B          is the apparent biological age determined in step (c3), and
mean(a)    is the mean chronological age of the statistical population used in the study underlying the present invention.

In this embodiment, establishing a multiple linear regression model for the determined biomarkers based on the data provided in the study underlying the present invention, and thus determining the parameters $r^2$, $b_1$ to $b_n$, and c, is not particularly limited and can be done with statistical methods known in the art. As an example, the package "stats" which is a component of the free software "R" can be used in this respect.
In another aspect of the present invention, any number n of the above biomarkers (i) to (xxviii) for females or of the above biomarkers (i) to (xxiii) for males can be used for the determination of the biological age of a human being, provided that n is at least 2, at least 3, at least 4, at least 5, at least 6, at least 7, at least 8, at least 9, or at least 10. In these embodiments, step (c) comprises the steps (c1) to (c4) as described above. In this context, it is emphasized that

the above parameter $r^2$ as determined from the multiple linear regression model established in step (c1) is a measure for the correlation of the biological age determined with a particular subset of biomarkers with the chronological age. Accordingly, the quality of any given subset of biomarkers, *i.e.* the ability of said subset to predict the biological age, can be determined. Thus, respective high quality subset of biomarkers can be identified and chosen for further use.

In this context, the above female and male biomarkers (i) to (x) have been identified in the study underlying the present invention to result in a particularly good correlation with chronological age and, thus, in a particularly good determination of the biological age. This is based on a definition of biological age as the expected chronological age given only biological information.

Therefore, in a particularly preferred embodiment of the present invention, the human being is female, the biomarkers (i) to (x) are determined in step (b1), and determining the biological age of the human being based on said biomarkers in step (c) comprises the steps of:

(c1) calculating an apparent biological age B according to formula (3)

$$B = \sum_{i=1}^{10} b_i x_i + c \qquad (3)$$

wherein

$x_1$ is the determined value of biomarker (i), preferably expressed as a percentage between 0 and 1,
$x_2$ is the determined value of biomarker (ii), preferably expressed as a percentage between 0 and 1,
$x_3$ is the determined value of biomarker (iii), preferably expressed as a percentage between 0 and 1,
$x_4$ is the determined value of biomarker (iv), preferably expressed as a percentage between 0 and 1,
$x_5$ is the determined value of biomarker (v), preferably expressed as a percentage between 0 and 1,
$x_6$ is the determined value of biomarker (vi), preferably expressed in g per dL,
$x_7$ is the determined value of biomarker (vii), preferably expressed in ng per mL,
$x_8$ is the determined value of biomarker (viii),
$x_9$ is the determined value of biomarker (ix), preferably expressed in mole per L,
$x_{10}$ is the determined value of biomarker (x), preferably expressed in mole per L; and

(c2) calculating the corrected biological age (Bioage) according to formula (2)

$$Bioage = \frac{B - mean(a)}{r^2} + mean(a) \qquad (2)$$

wherein

B is the apparent biological age determined in step (c1).

Further, by way of a multiple linear regression model that has been established for the above biomarkers (i) to (x), the above parameters $b_1$ to $b_{10}$ have been determined to have the following values:

$b_1$ is 44.85,
$b_2$ is 47.616,
$b_3$ is -25.457,
$b_4$ is 29.463,
$b_5$ is 13.19,
$b_6$ is -0.889,
$b_7$ is 0.025,
$b_8$ is 10.863,
$b_9$ is 0.1,
$b_{10}$ is 0.031,
c is -13.16, and
$r^2$ is 0.87.

Further, mean(a), *i.e.* the mean age of the test population examined in the study underlying the present invention, is 55.85 years.

Accordingly, in a preferred embodiment, the parameters $b_1$ to $b_{10}$, c, $r^2$, and mean(a) have the above values.

However, a person skilled in the art will understand that an increase of the test population, providing additional data for the correlation of each biomarker with chronological age, will subject the above parameters to variation. Therefore, it will be understood that the above parameters may vary for up to +/- 10%. Accordingly, the respective values in this range are intended to be encompassed in the present invention.

According to a particular embodiment,

$b_1$      is a number between 40.37 and 49.33,
$b_2$      is a number between 42.86 and 52.37,
$b_3$      is a number between -28.00 and -22.92,
$b_4$      is a number between 26.52 and 32.40,
$b_5$      is a number between 11.88 and 14.50,
$b_6$      is a number between -0.97 and -0.80,
$b_7$      is a number between 0.023 and 0.027,
$b_8$      is a number between 9.78 and 11.94
$b_9$      is a number between 0.09 and 0.11
$b_{10}$      is a number between 0.028 and 0.034,
c      is a number between -14.47 and -11.85
mean(a)      is a number between 50.27 and 61.43, and
$r^2$      is a number between 0.79 and 0.95.

In a corresponding particularly preferred embodiment, the human being is male, the biomarkers (i) to (x) are determined in step (b2), and determining the biological age of the human being based on said biomarkers in step (c) comprises the steps of:

(c1) calculating an apparent biological age B according to formula (3)

$$B = \sum_{i=1}^{10} b_i x_i + c \qquad\qquad (3)$$

wherein

$x_1$      is the determined value of biomarker (i), preferably expressed as a percentage between 0 and 1,
$x_2$      is the determined value of biomarker (ii), preferably expressed as a percentage between 0 and 1,
$x_3$      is the determined value of biomarker (iii), preferably expressed as a percentage between 0 and 1,
$x_4$      is the determined value of biomarker (iv), preferably expressed as a percentage between 0 and 1,
$x_5$      is the determined value of biomarker (v),
$x_6$      is the determined value of biomarker (vi),
$x_7$      is the determined value of biomarker (vii), preferably expressed in g per dL,
$x_8$      is the determined value of biomarker (viii), preferably expressed in mg per dL,
$x_9$      is the determined value of biomarker (ix), preferably expressed in mole per L,
$x_{10}$      is the determined value of biomarker (x), preferably expressed in mole per L, and

(c2) calculating the corrected biological age (Bioage) according to the formula (2)

$$Bioage = \frac{B - mean(a)}{r^2} + mean(a) \qquad\qquad (2)$$

wherein

B      is the apparent biological age determined in step (c1).

Further, by way of a multiple linear regression model that has been established for the above biomarkers (i) to (x), the above parameters $b_1$ to $b_{10}$ have been determined to have the following values:

$b_1$ is 36.481,
$b_2$ is 37.25,
$b_3$ is 47.234,
$b_4$ is 4.506,
$b_5$ is -0.172,
$b_6$ is 1.538,
$b_7$ is -1.044,
$b_8$ is 0.024,
$b_9$ is -3.131,
$b_{10}$ is 0.029,
$c$ is -3.858, and
$r^2$ is 0.86.

Further, mean(a), *i.e.* the mean age of the test population examined in the study underlying the present invention, is 55.85.

Accordingly, in a preferred embodiment, the parameters $b_1$ to $b_{10}$, $c$, $r^2$, and mean(a) have the above values.

However, as has been stated above, a person skilled in the art will understand that an increase of the test population, providing additional data for the correlation of each biomarker with chronological age, will subject the above parameters to variation. Therefore, it will be understood that the above parameters may vary for up to +/- 10%. Accordingly, the respective values in this range are intended to be encompassed in the present invention.

Thus, according to a particular embodiment,

$b_1$ is a number between 32.84 and 40.12,
$b_2$ is a number between 33.53 and 40.97,
$b_3$ is a number between 42.52 and 51.95,
$b_4$ is a number between 4.06 and 4.95,
$b_5$ is a number between -0.18 and -0.16,
$b_6$ is a number between 1.39 and 1.69,
$b_7$ is a number between -1.14 and -0.94,
$b_8$ is a number between 0.022 and 0.026,
$b_9$ is a number between -3.44 and -2.82,
$b_{10}$ is a number between 0.027 and 0.031,
$c$ is a number between -4.24 and -3.47; and mean(a) is a number between 50.27 and 61.43, and
$r^2$ is a number between 0.78 and 0.94.

The test population used in the study underlying the present invention had chronological ages between 35 and 74 years. Accordingly, the human being for which the biological age should be determined with a method of the present invention has preferably a chronological age between 30 and 80 years, more preferably between 35 and 74 years.

In further aspects, the present invention relates to the use of the above biomarkers for the determination of the biological age of a human being.

In particular, the present invention relates to the use of at least the following biomarkers (i) to (x) for the determination of the biological age of a female human being:

(i) cumulative level of cytosine methylation at gene positions ELOVL_2_CpG_11, ELOVL_2_CpG_12, ELOVL_2_CpG_13, and ELOVL_2_CpG_14 in blood cells;
(ii) cumulative level of cytosine methylation at gene positions ELOVL_2_CpG_15, ELOVL_2_CpG_16, and ELOVL_2_CpG_17 in blood cells;
(iii) cumulative level of cytosine methylation at gene positions FHL_2_CpG_11, and FHL_2_CpG_12 in blood cells;
(iv) cumulative level of cytosine methylation at gene positions FHL_2_CpG_13, FHL_2_CpG_14, and FHL_2_CpG_15 in blood cells;
(v) cumulative level of cytosine methylation at gene positions FHL_2_CpG_16 and FHL_2_CpG_17 in blood cells;
(vi) concentration of dehydroepiandrosterone sulfate in plasma;
(vii) concentration of ferritin in serum;
(viii) decadic logarithm of the ratio between the concentration of NGA2F glycan in serum and the concentration of NA2F glycan in serum;

(ix) concentration of alpha-tocopherol in plasma;

(x) concentration of cysteine in plasma.

In a preferred embodiment of this aspect, one or more of the following biomarkers (xi) to (xxviii) are used in addition to the biomarkers (i) to (x):

(xi) cumulative level of cytosine methylation at gene positions ELOVL_2_CpG_22, ELOVL_2_CpG_23, and ELOVL_2_CpG_24 in blood cells;
(xii) cumulative level of cytosine methylation at gene positions FHL_2_CpG_19, and FHL_2_CpG_20 in blood cells;
(xiii) concentration of NGA2FB glycan in serum;
(xiv) concentration of fibrinogen in plasma;
(xv) concentration of urea in plasma;
(xvi) concentration of glucose in serum;
(xvii) length of telomers in blood cells;
(xviii) concentration of triglycerides in serum;
(xix) concentration of immunoglobulin M (IgM) in serum;
(xx) concentration of adiponectin in serum;
(xxi) accumulated concentration of threonine and lactate in urine;
(xxii) concentration of homocysteine in plasma;
(xxiii) concentration of lycopene in plasma;
(xxiv) concentration of cholesterol in serum;
(xxv) concentration of glycosylated hemoglobin (HbA$_{1c}$) in whole blood;
(xxvi) concentration of glycine in urine;
(xxvii) number of T cell receptor excision circles (Trec) in 100,000 T cells from whole blood;
(xxviii) concentration of creatinine in urine.

However, in another preferred embodiment of this aspect, only the above biomarkers (i) to (x) are used.
In another aspect, the present invention relates to the use of at least the following biomarkers (i) to (x) for the determination of the biological age of a male human being:

(i) cumulative level of cytosine methylation at gene positions ELOVL_2_CpG_11, ELOVL_2_CpG_12, ELOVL_2_CpG_13, and ELOVL_2_CpG_14 in blood cells;
(ii) cumulative level of cytosine methylation at gene positions ELOVL_2_CpG_15, ELOVL_2_CpG_16, and ELOVL_2_CpG_17 in blood cells;
(iii) cumulative level of cytosine methylation at gene positions FHL_2_CpG_13, FHL_2_CpG_14, and FHL_2_CpG_15 in blood cells;
(iv) level of cytosine methylation at gene position FHL_2_CpG_18;
(v) concentration of creatinine in urine;
(vi) ratio of the number of CD3$^+$CD4$^+$ cells in whole blood to the number of CD8$^+$ cells in whole blood;
(vii) concentration of dehydroepiandrosterone sulfate in plasma;
(viii) concentration of alpha-2-macroglubulin in serum;
(ix) concentration of lycopene in plasma;
(x) concentration of cysteine in plasma.

In a preferred embodiment of this aspect, one or more of the following biomarkers (xi) to (xxiii) are used in addition to the biomarkers (i) to (x):

(xi) cumulative level of cytosine methylation at gene positions FHL_2_CpG_19, and FHL_2_CpG_20 in blood cells;
(xii) number of T cell receptor excision circles (Trec) in 100,000 T cells from whole blood;
(xiii) decadic logarithm of the ratio between the concentration of NGA2F glycan in serum and the concentration of NA2F glycan in serum;
(xiv) concentration of prostate specific antigen (PSA) in serum;
(xv) concentration of fibrinogen in plasma;
(xvi) concentration of albumin in plasma;
(xvii) concentration of glucose in serum;
(xviii) concentration of NGA2FB glycan in serum;
(xix) ratio of the concentration of Cu in plasma to the concentration of Zn in plasma;
(xx) ratio of the number of CD16$^+$CD56$^+$ cells in whole blood to the number of CD45$^+$ cells in whole blood;

(xxi) concentration of antibodies directed against *Clostridium tetani* in plasma;

(xxii) ratio of the number of CD3⁺CD8⁺ cells in whole blood to the number of CD45⁺ cells in whole blood;

(xxiii) concentration of beta-hydroxyisovalerate in urine.

However, in another preferred embodiment of this aspect, only the above biomarkers (i) to (x) are used.

Finally, in another preferred embodiment, the present invention relates to the use of any number n of the above biomarkers (i) to (xxviii) for females or of the above biomarkers (i) to (xxiii) for males for the determination of the biological age of a human being, provided that n is at least 2, at least 3, at least 4, at least 5, at least 6, at least 7, at least 8, at least 9, or at least 10.

The purpose of the present invention is to provide a valid set of biomarkers of aging in humans that is based on biochemical and molecular analyses of blood and its component or urine, enabling the determination of the biological age of the individual. This can serve as a valuable diagnostic tool for Preventive Medicine by enabling identification of healthy persons whose aging process is accelerated and who are thus likely to be affected by typical age-related diseases at relatively young chronological age. Accordingly, a diagnostic tool is provided for use in all fields wherein an individual's biological age is a valuable information, such as e.g. individual health care, health care systems, insurances and others.

In the following, the biomarkers used in the present invention are described. Table 1 below provides an overview over said biomarkers, assigning a running number (#) to each biomarker, giving the short name for each biomarker as used herein, providing a reference to the numbering in small roman numerals as used in the claims and the above description for female and male biomarkers (#(f) and #(m)), respectively, and providing information as to where in the Figures data concerning the correlation with chronological age of the respective biomarkers is shown.

Further, Table 2 below provides detailed information concerning the correlation of the respective biomarkers with chronological age, in particular the size of the test population (count), and the non-parametric correlation coefficient (rnp).

Table 1: Overview of the biomarkers used in the present invention.

| # | short name | #(f) | #(m) | Fig. |
|---|---|---|---|---|
| 1 | ELOVL_2_CpG_11, 12, 13, 14 | (i) | (i) | 1 |
| 2 | ELOVL_2_CpG_15, 16, 17 | (ii) | (ii) | 2 |
| 3 | ELOVL_2_CpG_22, 23, 24 | (xi) | - | 3 |
| 4 | FHL_2_CpG_11,12 | (iii) | - | 4 |
| 5 | FHL_2_CpG_13, 14, 15 | (iv) | (iii) | 5 |
| 6 | FHL_2_CpG_16, 17 | (v) | - | 6 |
| 7 | FHL_2_CpG_18 | - | (iv) | 7 |
| 8 | FHL_2_CpG_19,20 | (xii) | (xi) | 8 |
| 9 | dehydroepiandrosterone sulfate | (vi) | (vii) | 9 |
| 10 | ferritin | (vii) | - | 10 |
| 11 | S_log_p1_p6 | (viii) | (xiii) | 11 |
| 12 | plasma alpha.tocopherole | (ix) | - | 12 |
| 13 | plasma cysteine | (x) | (x) | 13 |
| 14 | creatinine.urine | (xxviii) | (v) | 14 |
| 15 | alpha2 macroglobulin | - | (viii) | 15 |
| 16 | plasma lycopene | (xxiii) | (ix) | 16 |
| 17 | S_p2_n_glycan | (xiii) | (xviii) | 17 |
| 18 | fibrinogen | (xiv) | (xv) | 18 |
| 19 | urea | (xv) | - | 19 |
| 20 | serum glucose | (xvi) | (xvii) | 20 |
| 21 | telomere length | (xvii) | - | 21 |

(continued)

| # | short name | #(f) | #(m) | Fig. |
|---|---|---|---|---|
| 22 | triglycerides | (xviii) | - | 22 |
| 23 | immunoglobulin M | (xix) | - | 23 |
| 24 | adiponectin | (xx) | - | 24 |
| 25 | threonine/lactate | (xxi) | - | 25 |
| 26 | homocysteine | (xxii) | - | 26 |
| 27 | cholesterol | (xxiv) | - | 27 |
| 28 | glycosylated hemoglobin A1 C | (xxv) | - | 28 |
| 29 | glycine in urine | (xxvi) | - | 29 |
| 30 | Trec | (xxvii) | (xii) | 30 |
| **#** | **short name** | **#c (f)** | **#c (m)** | **Fig.** |
| 31 | prostate specific antigen | - | (xiv) | 31 |
| 32 | albumin | - | (xvi) | 32 |
| 33 | plasma Cu/Zn | - | (xix) | 33 |
| 34 | $CD16^+CD56^+/CD45^+$ | - | (xx) | 34 |
| 35 | tetanus IgG antibodies | - | (xxi) | 35 |
| 36 | $CD3^+CD8^+/CD45^+$ | - | (xxii) | 36 |
| 37 | beta hydroxyisovalerate | - | (xxiii) | 37 |
| 38 | $CD3^+CD4^+/CD8^+$ | - | (vi) | 38 |

#: running number
short name: short name of the biomarker
#(f): numbering as used in the claims for female biomarkers
#(m): numbering as used in the claims for male biomarkers
Fig.: biomarker correlation data shown in Figure

Table 2: Correlation data with chronological age

| # | short name | count female | rnp female | count male | rnp male |
|---|---|---|---|---|---|
| 1 | ELOVL_2_CpG_11, 12, 13, 14 | 1066 | 0.779 | 1006 | 0.752 |
| 2 | ELOVL_2_CpG_15, 16, 17 | 1119 | 0.754 | 1053 | 0.723 |
| 3 | ELOVL_2_CpG_22, 23, 24 | 1077 | 0.539 | 1012 | 0.446 |
| 4 | FHL_2_CpG_11, 12 | 1121 | 0.443 | 1054 | 0.443 |
| 5 | FHL_2_CpG_13, 14, 15 | 1121 | 0.526 | 1055 | 0.532 |
| 6 | FHL_2_CpG_16,17 | 1077 | 0.577 | 1012 | 0.508 |
| 7 | FHL_2_CpG_18 | 851 | 0.361 | 826 | 0.347 |
| 8 | FHL_2_CpG_19,20 | 441 | 0.591 | 399 | 0.567 |
| 9 | dehydroepiandrosterone sulfate | 1139 | -0.480 | 1066 | -0.547 |
| 10 | ferritin | 1096 | 0.458 | 984 | 0.089 |
| 11 | S_log_p1_p6 | 1014 | 0.605 | 860 | 0.293 |
| 12 | plasma alpha.tocopherole | 1139 | 0.308 | 1068 | 0.093 |
| 13 | plasma cysteine | 1138 | 0.276 | 1069 | 0.195 |

(continued)

| # | short name | count female | rnp female | count male | rnp male |
|---|---|---|---|---|---|
| 14 | creatinine.urine | 946 | -0.204 | 913 | -0.235 |
| 15 | alpha2 macroglobulin | 1091 | 0.116 | 974 | 0.288 |
| 16 | plasma lycopene | 1139 | -0.230 | 1068 | -0.344 |
| 17 | S_p2_n_glycan | 1014 | 0.503 | 860 | 0.245 |
| 18 | fibrinogen | 1137 | 0.362 | 1062 | 0.251 |
| 19 | urea | 1137 | 0.338 | 1063 | 0.148 |
| 20 | serum glucose | 1095 | 0.313 | 985 | 0.246 |
| 21 | telomere length | 192 | -0.302 | 128 | -0.096 |
| 22 | triglycerides | 1094 | 0.296 | 985 | -0.028 |
| 23 | immunoglobulin M | 1091 | -0.261 | 975 | -0.082 |
| 24 | adiponectin | 325 | 0.261 | 234 | 0.126 |
| 25 | threonine/lactate | 946 | -0.235 | 913 | -0.093 |
| 26 | homocysteine | 1137 | 0.232 | 1063 | 0.071 |
| 27 | cholesterol | 1095 | 0.226 | 985 | -0.081 |
| 28 | glycosylated hemoglobin A1 C | 1052 | 0.219 | 929 | 0.137 |
| 29 | glycine in urine | 946 | -0.218 | 913 | -0.102 |
| 30 | Trec | 258 | -0.215 | 219 | -0.342 |
| 31 | prostate specific antigen | 1091 | -0.146 | 960 | 0.271 |
| 32 | albumin | 1137 | -0.073 | 1063 | -0.25 |
| 33 | plasma Cu/Zn | 1022 | 0.111 | 877 | 0.228 |
| 34 | $CD16^{+}CD56^{+}/CD45^{+}$ | 751 | 0.136 | 712 | 0.223 |
| 35 | tetanus IgG antibodies | 1133 | -0.22 | 1061 | -0.216 |
| 36 | $CD3^{+}CD8^{+}/CD45^{+}$ | 752 | -0.182 | 722 | -0.215 |
| 37 | beta hydroxyisovalerate | 946 | -0.157 | 913 | -0.205 |
| 38 | $CD3^{+}CD4^{+}/CD8^{+}$ | 752 | 0.152 | 728 | 0.181 |

#: running number
short name: short name of the biomarker
count: number of subjects
rnp: non-parametric coefficient of correlation

The following provides a detailed description of the biomarkers.

1. ELOVL 2 CpG 11, 12, 13, 14

This biomarker is defined as the cumulative level of cytosine methylation at gene positions ELOVL_2_CpG_11, ELOVL_2_CpG_12, ELOVL_2_CpG_13, and ELOVL_2_CpG_14. ELOVL_2 is a gene belonging to the ELOVL (Elongation of very long chain fatty acids) gene family whose gene products control the synthesis of very-long-chain fatty acids. The ELOVL_2 gene has a number of CpG motifs in which the cytosine can be methylated or unmethylated. For this biomarker, the methylation of the CpG motifs ELOVL_2_CpG_11, ELOVL_2_CpG_12, ELOVL_2_CpG_13, and ELOVL_2_CpG_14 is determined in blood cells in a cumulative manner over all measured cells. This marker is given as a percentage between 0 and 1, i.e. when e.g. none of the above CpG motifs are methylated, the value is 0, when e.g. 50% of the above CpG motifs are methylated, the value is 0.5, and when e.g. all of the above CpG motifs are methylated, the value is 1. Methods for the determination of the level of cytosine methylation at certain gene positions are not particularly limited and are known in the art.

## 2. ELOVL 2 CpG 15, 16, 17

In an analogous manner to the above biomarker ELVOL_2_CpG_11, 12, 13, 14, this biomarker is defined as the cumulative level of cytosine methylation at gene positions ELOVL_2_CpG_15, ELOVL_2_CpG_16, and ELOVL_2_CpG_17.

## 3. ELOVL 2 CpG 22, 23, 24

In an analogous manner to the above biomarker ELVOL_2_CpG_11, 12, 13, 14, this biomarker is defined as the cumulative level of cytosine methylation at gene positions ELOVL_2_CpG_22, ELOVL_2_CpG_23, and ELOVL_2_CpG_24.

## 4. FHL 2 CpG 11, 12

This biomarker is defined as the cumulative level of cytosine methylation at gene positions FHL_2_CpG_11 and FHL_2_CpG_12. FHL_2 is the gene encoding the "Four and a half LIM domains protein 2" (FHL2) which contains highly conserved double zinc finger motifs called the LIM domain. FHL2 has been shown to interact with a number of intracellular protein factors. Cumulative cytosine methylation is determined in an analogous manner as described for the above biomarker ELOVL_2_CpG_11, 12, 13, 14.

## 5. FHL 2 CpG 13, 14, 15

In an analogous manner to the above biomarker FHL_2_CpG_11, 12, this biomarker is defined as the cumulative level of cytosine methylation at gene positions FHL_2_CpG_13, FHL_2_CpG_14, and FHL_2_CpG_15.

## 6. FHL 2 CpG 16, 17

In an analogous manner to the above biomarker FHL_2_CpG_11, 12, this biomarker is defined as the cumulative level of cytosine methylation at gene positions FHL_2_CpG_16, and FHL_2_CpG_17.

## 7. FHL 2 CpG 18

In an analogous manner to the above biomarker FHL_2_CpG_11, 12, this biomarker is defined as the cumulative level of cytosine methylation at gene position FHL_2_CpG_18.

## 8. FHL 2 CpG 19, 20

In an analogous manner to the above biomarker FHL_2_CpG_11, 12, this biomarker is defined as the cumulative level of cytosine methylation at gene positions FHL_2_CpG_19, and FHL_2_CpG_20.

## 9. Dehydroepiandrosterone sulfate

This biomarker is defined as the concentration of dehydroepiandrosterone sulfate in plasma. Dehydroepiandrosterone is an important endogenous steroid hormone and the most abundant steroid in humans. It is produced in the adrenal glands, the gonads and the brain and functions as a metabolic intermediate in the biosynthesis of the androgen and estrogen sex steroids. Dehydroepiandrosterone sulfate is produced exclusively in the adrenal glands via the sulfotransferases SULT1A1 and SULT1E1. Preferably, the value of this biomarker is given in µg per dL. Methods for the determination of the concentration of dehydroepiandrosterone sulfate in plasma are not particularly limited and are known in the art.

## 10. Ferritin

This biomarker is defined as the concentration of ferritin in serum. Ferritin is an ubiquitous intracellular protein that stores and releases iron in a controlled fashion. Preferably, the value of this biomarker is given in ng per mL. Methods for the determination of the concentration of ferritin in serum are not particularly limited and are known in the art.

## 11. S log p1 p6

This biomarker is defined as the decadic logarithm of the ratio between the concentration of NGA2F glycan in serum and the concentration of NA2F glycan in serum. NGA2F glycan is an agalactosylated core-$\alpha$-1,6-fucosylated biantennary oligosaccharide, whereas NA2F glycan is a bigalactosylated core-$\alpha$-1,6-fucosylated biantennary oligosaccharide. Methods for the determination of the concentration of NGA2F glycan in serum and the concentration of NA2F glycan in serum are not particularly limited and are known in the art.

## 12. Plasma alpha.tocopherole

This biomarker is defined as the concentration of alpha-tocopherole in plasma. Alpha-tocopherole is a form of vitamin E. Preferably, the value of this biomarker is given in $\mu$mole per L. Methods for the determination of the concentration of alpha-tocopherole in plasma are not particularly limited and are known in the art.

## 13. Plasma cysteine

This biomarker is defined as the concentration of cysteine in plasma. Preferably, the value of this biomarker is given in $\mu$mole per L. Methods for the determination of the concentration of cysteine in plasma are not particularly limited and are known in the art.

## 14. Creatinine.urine

This biomarker is defined as the concentration of creatinine in urine. Methods for the determination of the concentration of creatinine in urine are not particularly limited and are known in the art. As an example, this marker can be determined by NMR-based techniques.

## 15. Alpha2 macroglobulin

This biomarker is defined as the concentration of alpha-2-macroglobulin in serum. Alpha-2-macroglobulin is the largest major non-immunoglobulin protein in plasma. It acts as an antiprotease and inhibitor of both fibrinolysis and coagulation. Preferably, the value of this biomarker is given in mg per dL. Methods for the determination of the concentration of alpha-2-macroglobulin in serum are not particularly limited and are known in the art.

## 16. Plasma lycopene

This biomarker is defined as the concentration of lycopene in plasma. Lycopene is a carotene and carotinoid pigment and a phytochemical found in tomatoes. It is not an essential nutrient for humans, has antioxidant activity and is transported in the blood by various lipoproteins. Lycopene accumulates in the liver, adrenal glands and testes. It is a potential agent for the prevention of some types of cancer. Preferably, the value of this biomarker is given in $\mu$mole per L. Methods for the determination of the concentration of lycopene in plasma are not particularly limited and are known in the art.

## 17. S p2 n glycan

This biomarker is defined as the concentration of NGA2FB glycan in serum. NGA2FB glycan is an agalactosylated core-$\alpha$-1,6-fucosylated bisecting biantennary oligosaccharide. Preferably, the value of this biomarker is given as a percentage of all glycan signals. Methods for the determination of the concentration of NGA2FB glycan in serum are not particularly limited and are known in the art.

## 18. Fibrinogen

This biomarker is defined as the concentration of fibrinogen in plasma. Fibrinogen is a soluble plasma glycoprotein that is synthesized in the liver and is converted by thrombin to fibrin during blood coagulation. Fibrin plays a key role in the inflammatory response and development of rheumatoid arthritis. Preferably, the value of this biomarker is given in mg per mL. Methods for the determination of the concentration of fibrinogen in plasma are not particularly limited and are known in the art.

### 19. Urea

This biomarker is defined as the concentration of urea in plasma. Preferably, the value of this biomarker is given in mmole per L. Methods for the determination of the concentration of urea in plasma are not particularly limited and are known in the art.

### 20. Serum glucose

This biomarker is defined as the concentration of glucose in serum. Preferably, the value of this biomarker is given in mmole per L. Methods for the determination of the concentration of glucose in serum are not particularly limited and are known in the art.

### 21. Telomer length

This biomarker is defined as the length of telomers in blood cells. A telomere is a region of repetitive nucleotide sequences at each end of a chromatid, which protects the end of the chromosome from deterioration or from fusion with neighboring chromosomes. Telomere regions deter the degradation of genes near the ends of chromosomes by allowing chromosome ends to shorten, which necessarily occurs during chromosome replication. Over time, due to each cell division, the telomere ends become shorter. Preferably, the value of this biomarker is given in kilobases (kb). Methods for the determination of the length of telomers in blood cells are not particularly limited and are known in the art.

### 22. Triglycerides

This biomarker is defined as the concentration of triglycerides in serum. Preferably, the value of this biomarker is given in mmole per L. Methods for the determination of the concentration of triglycerides in serum are not particularly limited and are known in the art.

### 23. Immunoglobulin M

This biomarker is defined as the concentration of immunoglobulin M (IgM) in serum. Preferably, the value of this biomarker is given in g per L. Methods for the determination of the concentration of immunoglobulin M (IgM) in serum are not particularly limited and are known in the art.

### 24. Adiponectin

This biomarker is defined as the concentration of adiponectin in serum. Adiponectin is a protein hormone that modulates glucose regulation and fatty acid oxidation. It is secreted from adipose tissue into the bloodstream and is very abundant. Adiponectin levels inversely correlate with body fat percentage in adults and plays a role in the suppression of metabolic derangements that may result in type 2 diabetes, obesity, atherosclerosis or non-alcoholic fatty liver disease. Preferably, the value of this biomarker is given in ng per mL. Methods for the determination of the concentration of adiponectin in serum are not particularly limited and are known in the art.

### 25. Threonine/Lactate

This biomarker is defined as the accumulated, *i.e.* added, concentration of threonine and lactate in urine. Methods for the determination of the concentrations of threonine and lactate in urine are not particularly limited and are known in the art. As an example, this marker can be determined by NMR-based techniques.

### 26. Homocysteine

This biomarker is defined as the concentration of homocysteine in plasma. Homocysteine is a non-proteinogenic amino acid high levels of which have been linked to cardiovascular disease. Preferably, the value of this biomarker is given in $\mu$mole per L. Methods for the determination of the concentration of homocysteine in plasma are not particularly limited and are known in the art.

### 27. Cholesterol

This biomarker is defined as the concentration of cholesterol in serum. Cholesterol is an essential structural component of mammalian cell membranes and is required to establish proper membrane permeability and fluidity and is thus manufactured by every cell. In addition to its importance within cells, cholesterol also serves as a precursor for the biosynthesis of steroid hormones, bile acids, and vitamin D. Preferably, the value of this biomarker is given in mmole per L. Methods for the determination of the concentration of cholesterol in serum are not particularly limited and are known in the art.

28. Glycosylated hemoglobin $A_{1C}$

This biomarker is defined as the concentration of glycosylated hemoglobin ($HbA_{1c}$) in whole blood. $HbA_{1c}$ is formed in a non-enzymatic glycation pathway by hemoglobin's exposure to plasma glucose. Normal levels of glucose produce a normal amount of $HbA_{1c}$. As the average amount of plasma glucose increases, the fraction of $HbA_{1c}$ increases in a predictable way. Preferably, the value of this biomarker is given in percent of total hemoglobin. Methods for the determination of the concentration of glycosylated hemoglobin ($HbA_{1c}$) in whole blood are not particularly limited and are known in the art.

29. Glycine

This biomarker is defined as the concentration of glycine in urine. Methods for the determination of the concentration of glycine in urine are not particularly limited and are known in the art. As an example, this marker can be determined by NMR-based techniques.

30. Trec

This biomarker is defined as the number of T cell receptor excision circles (Trec) in 100,000 T cells from whole blood. Trecs are small circles of DNA created in T cells during their passage through the thymus as they rearrange their T cell receptor genes. Methods for the determination of the number of T cell receptor excision circles (Trec) in T cells from whole blood are not particularly limited and are known in the art.

31. Prostate specific antigen

This biomarker is defined as the concentration of prostate specific antigen (PSA) in serum. PSA a member of the kallikrein-related peptidase family and is secreted by the epithelial cells of the prostate gland. PSA is produced for the ejaculate, where it liquefies semen in the seminal coagulum and allows sperm to swim freely. It is also believed to be instrumental in dissolving cervical mucus, allowing the entry of sperm into the uterus. PSA is present in small quantities in the serum of men with healthy prostates, but is often elevated in the presence of prostate cancer or other prostate disorders. Preferably, the value of this biomarker is given in ng per mL. Methods for the determination of the concentration of prostate specific antigen (PSA) in serum are not particularly limited and are known in the art.

32. Albumin

This biomarker is defined as the concentration of albumin in plasma. Albumin is the main protein of human plasma. It binds water, cations, fatty acids, hormones, bilirubin, thyroxine (T4) and drugs (including barbiturates). Its main function is to regulate the colloidal osmotic pressure of blood. Preferably, the values of this biomarker is given in g per L. Methods for the determination of the concentration of albumin in plasma are not particularly limited and are known in the art.

33. Plasma Cu/Zn

This biomarker is defined as the ratio of the concentration of Cu in plasma to the concentration of Zn in plasma. Methods for the determination of the concentration of Cu in plasma and the concentration of Zn in plasma are not particularly limited and are known in the art.

34. CD16$^+$CD56$^+$/CD45$^+$

This biomarker is defined as the ratio of the number of CD16$^+$CD56$^+$ cells in whole blood to the number of CD45$^+$ cells in whole blood. CD16 and CD56 are markers of natural killer (NK) cells, whereas CD45 is a common leukocyte marker. Methods for the determination of the number of CD16$^+$CD56$^+$ cells in whole blood and of the number of CD45$^+$ cells in whole blood are not particularly limited and are known in the art.

35. Tetanus IgG antibodies

This biomarker is defined as the concentration of IgG antibodies directed against *Clostridium tetani* in plasma. Preferably, the value of this biomarker is given in IU (international units) per mL. Methods for the determination of the concentration of IgG antibodies directed against *Clostridium tetani* in plasma are not particularly limited and are known in the art.

36. CD3$^+$CD8$^+$/CD45$^+$

This biomarker is defined as the ratio of the number of CD3$^+$CD8$^+$ cells in whole blood to the number of CD45$^+$ cells in whole blood. CD3 is a T cell marker and CD8 is a marker for cytotoxic T cells. CD45 is a common leukocyte marker. Methods for the determination of the number of CD3$^+$CD8$^+$ cells in whole blood and of the number of CD45$^+$ cells in whole blood are not particularly limited and are known in the art.

37. Beta hydroxyisovalerate

This biomarker is defined as the concentration of beta-hydroxyisovalerate in urine. Methods for the determination of the concentration of beta-hydroxyisovalerate in urine are not particularly limited and are known in the art. As an example, this marker can be determined by NMR-based techniques.

38. CD3$^+$CD4$^+$/CD8$^+$

This biomarker is defined as the ratio of the number of CD3$^+$CD4$^+$ cells in whole blood to the number of CD8$^+$ cells in whole blood. CD3 is a T cell marker and CD4 is a marker for T helper cells, whereas CD8 is a marker for cytotoxic T cells. Methods for the determination of the number of CD3$^+$CD4$^+$ cells in whole blood and of the number of CD8$^+$ cells in whole blood are not particularly limited and are known in the art.

The present invention provides methods for the determination of the biological age for women or men, respectively, which is based on a set of at least ten different molecular and clinical chemistry biomarkers that have been assessed in blood, blood components or urine. Selection of these parameters and their weighting was done in such as to way as to maximize the correlation coefficient between the biological age and the chronological age in the population.

The methods of the present invention advantageously reflect biological mechanisms known to play important roles in the aging of cells or tissues, including epigenetic changes, changes in N-glycan profiles, hormonal changes and changes in metabolism. Said methods are based on a set of at least ten different biomarkers, which obviously reflects the aging process better than any single marker on its own. Further, the biomarkers used in the present invention are solely based on analyses on blood or urine and therefore do not require physical examination of the subjects nor their cooperation nor motivation to give maximum performance in physiological assessment (e.g. lung function). This also reduces labor costs.

The figures show:

Figures 1 to 38:

These figures show the correlation of the respective biomarkers given in the top line of each figure with chronological age. Data for males is shown in the left panel, data for females in the right panel. In the graphs shown in the lower row, the 1% quantile is hidden, *i.e.* the highest and lowest 1% of datapoints with respect to the y-axis are excluded. The x-axis shows chronological age in years. The y-axis shows the respective biomarker values. The number of test subjects, the function of the linear regression, and the parametric (rp) and non-parametric (rnp) correlation coefficients are shown.

Figure 39:

Correlation between chronological age and biological age as determined according to the present invention using the female and male biomarkers (i) to (x), respectively, showing results from RASIG population (blue circles) and GeHA offspring subjects (red circles). count: number of test subjects; cor: correlation coefficient; mean rel dif: mean relative difference between the groups.

The present invention will be further illustrated in the following examples without being limited thereto.

**Examples**

Example 1:

Determination of the biological age of females

For ten exemplary female test subjects, the above biomarkers (i) to (x) (*i.e.* the biomarkers ELOVL_2_CpG_11, 12, 13, 14; ELOVL_2_CpG_15, 16, 17; FHL_2_CpG_11, 12; FHL_2_CpG_13, 14, 15; FHL_2_CpG_16, 17; dehydroepiandrosterone sulfate; ferritin; S_log_p1_p6; plasma alpha.tocopherole; and plasma cysteine) were determined. Based on the respective data, the biological age of the test subjects was determined according to the method of the present invention. Table 3 below shows test subject ID, and the chronological age (A) and biological age (B) of the test subject.

Table 3

| ID | A | B |
| --- | --- | --- |
| 95 | 61.105 | 60.372 |
| 97 | 67.032 | 79.909 |
| 77 | 67.687 | 74.668 |
| 104 | 48.461 | 46.446 |
| 105 | 70.408 | 59.619 |
| 350 | 38.75 | 32.993 |
| 109 | 67.618 | 60.429 |
| 107 | 48.067 | 44.747 |
| 108 | 48.675 | 43.208 |
| 106 | 65.277 | 79.714 |

Example 2:

Determination of the biological age of males

For ten exemplary male test subjects, the above biomarkers (i) to (x) (*i.e.* the biomarkers ELOVL_2_CpG_11, 12, 13, 14; ELOVL_2_CpG_15, 16, 17; FHL_2_CpG_13, 14, 15; FHL_2_CpG_18; creatinine.urine; $CD3^+CD4^+/CD8^+$; dehydroepiandrosterone sulfate; alpha2 macroglobulin; plasma lycopene; and plasma cysteine) were determined. Based on the respective data, the biological age of the test subjects was determined according to the method of the present invention. Table 4 below shows test subject ID, and the chronological age (A) and biological age (B) of the test subject.

Table 4

| ID | A | B |
| --- | --- | --- |
| 96 | 53.14 | 40.581 |
| 98 | 69.329 | 69.466 |
| 166 | 63.749 | 67.807 |
| 268 | 56.048 | 49.27 |
| 174 | 73.951 | 73.015 |
| 183 | 73.464 | 59.655 |
| 315 | 65.827 | 68.023 |
| 316 | 67.886 | 66.186 |
| 519 | 59.968 | 62.621 |

(continued)

| ID | A | B |
|---|---|---|
| 523 | 49.989 | 54.134 |

Example 3:

Detailed determination of the biological age

For a female individual, the above biomarkers (i) to (x) (*i.e.* the biomarkers ELOVL_2_CpG_11, 12, 13, 14; ELOVL_2_CpG_15, 16, 17; FHL_2_CpG_11, 12; FHL_2_CpG_13, 14, 15; FHL_2_CpG_16, 17; dehydroepiandrosterone sulfate; ferritin; S_log_p1_p6; plasma alpha.tocopherole; and plasma cysteine) were determined, wherein the respective values are given in Table 5 below.

Table 5

| # | Biomarker | Value |
|---|---|---|
| i | ELOVL_2_CpG_11, 12, 13, 14 | 0.63 |
| ii | ELOVL_2_CpG_15, 16, 17 | 0.56 |
| iii | FHL_2_CpG_11, 12 | 0.16 |
| iv | FHL_2_CpG_13, 14, 15 | 0.29 |
| v | FHL_2_CpG_16, 17 | 0.43 |
| vi | dehydroepiandrosterone sulfate [$\mu$g/dL] | 1.08 |
| vii | ferritin [ng/mL] | 146.1 |
| viii | S_log_p1_p6 | -0.445 |
| ix | plasma alpha.tocopherole [$\mu$mole/L] | 35.33 |
| x | plasma cysteine [$\mu$mole/L] | 195.5 |

According to the formula of the present invention (cf. pages 13 and 14, *supra*), the biological age of the individual was calculated to be 58.82 years. The chronological age of the individual at that time was 61.11 years.

Example 4:

Validation by comparison between RASIG population and GeHA offspring

In order validate the methods of the present invention and to determine whether said methods provide reasonable results in terms of biological age, results from the RASIG population, *i.e.* the test population used in the study underlying the present invention, were compared with results from GeHA offspring subjects. Since GeHA offspring subjects are born from a long-living parent, belong to a family with long living sibling(s), and for genetic reasons are expected to age at a slower rate, GeHA offspring should show lower biological ages as the RASIG population.

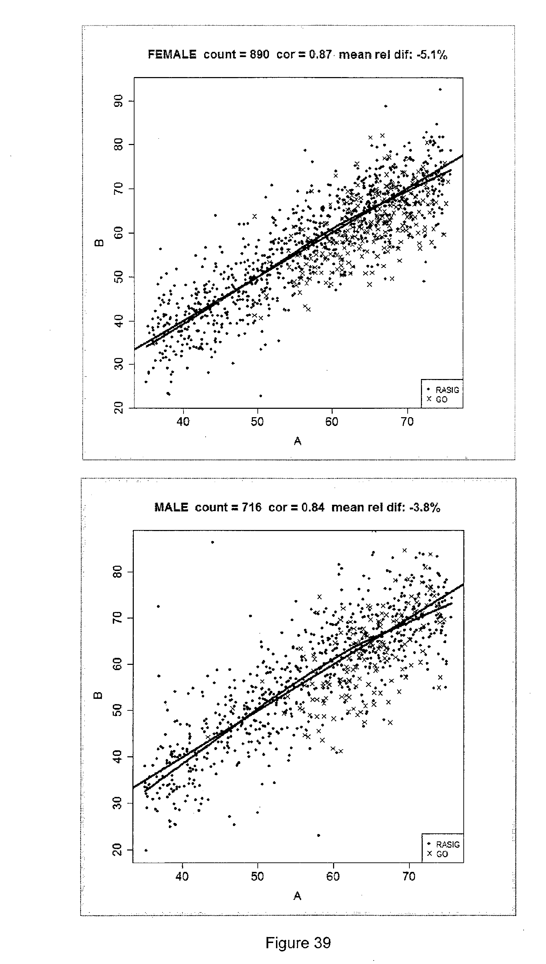

Figure 39 shows the respective correlations between chronological age and biological age for female/male RASIG test subjects (blue circles) and GeHA offspring subjects (red circles). Biological age was determined according to the present invention while using biomarkers (i) to (x) for females and males, respectively. Importantly, GeHA offspring subjects displayed a lower biological age than RASIG subjects of the same chronological age, in perfect agreement with other studies showing that the aging process in GeHA offspring in the middle age range is slowed down, compared to the general population. In particular, for females the mean relative difference between the two groups was -5.1%, and males -5.6%. Thus, the methods of the present invention appear to provide reasonable data with respective to biological age.

**Claims**

**1.**    A method for the determination of the biological age of a human being, comprising the steps of:

(a) providing one or more biological samples, selected from the group consisting of whole blood, serum, plasma, and urine, from the human being,

(b1) in case the human being is female, determining at least the following biomarkers (i) to (x) in the respective biological samples, wherein the necessary biological samples are provided in step (a):

(i) cumulative level of cytosine methylation at gene positions ELOVL_2_CpG_11, ELOVL_2_CpG_12, ELOVL_2_CpG_13, and ELOVL_2_CpG_14 in blood cells;
(ii) cumulative level of cytosine methylation at gene positions ELOVL_2_CpG_15, ELOVL_2_CpG_16, and ELOVL_2_CpG_17 in blood cells;
(iii) cumulative level of cytosine methylation at gene positions FHL_2_CpG_11, and FHL_2_CpG_12 in blood cells;
(iv) cumulative level of cytosine methylation at gene positions FHL_2_CpG_13, FHL_2_CpG_14, and FHL_2_CpG_15 in blood cells;
(v) cumulative level of cytosine methylation at gene positions FHL_2_CpG_16 and FHL_2_CpG_17 in blood cells;
(vi) concentration of dehydroepiandrosterone sulfate in plasma;
(vii) concentration of ferritin in serum;
(viii) decadic logarithm of the ratio between the concentration of NGA2F glycan in serum and the concentration of NA2F glycan in serum;
(ix) concentration of alpha-tocopherol in plasma;
(x) concentration of cysteine in plasma; or

(b2) in case the human being is male, determining at least the following biomarkers (i) to (x) in the respective biological samples, wherein the necessary biological samples are provided in step (a):

(i) cumulative level of cytosine methylation at gene positions ELOVL_2_CpG_11, ELOVL_2 CpG_12, ELOVL_2_CpG_13, and ELOVL_2_CpG_14 in blood cells;
(ii) cumulative level of cytosine methylation at gene positions ELOVL_2_CpG_15, ELOVL_2_CpG_16, and ELOVL_2_CpG_17 in blood cells;
(iii) cumulative level of cytosine methylation at gene positions FHL_2_CpG_13, FHL_2_CpG_14, and FHL_2_CpG_15 in blood cells;
(iv) cumulative level of cytosine methylation at gene position FHL_2_CpG_18;
(v) concentration of creatinine in urine;
(vi) ratio of the number of CD3$^+$CD4$^+$ cells in whole blood to the number of CD8$^+$ cells in whole blood;
(vii) concentration of dehydroepiandrosterone sulfate in plasma;
(viii) concentration of alpha-2-macroglubulin in serum;
(ix) concentration of lycopene in plasma;
(x) concentration of cysteine in plasma; and

(c) determining the biological age of the human being based on the biomarkers determined in step (b1)/(b2).

2. The method according to claim 1, wherein the human being is female, in step (b1) one or more of the following biomarkers (xi) to (xxviii) are determined in addition to the biomarkers (i) to (x), and the necessary biological samples are provided in step (a):

(xi) cumulative level of cytosine methylation at gene positions ELOVL_2_CpG_22, ELOVL_2_CpG_23, and ELOVL_2_CpG_24 in blood cells;
(xii) cumulative level of cytosine methylation at gene positions FHL_2_CpG_19, and FHL_2_CpG_20 in blood cells;
(xiii) concentration of NGA2FB glycan in serum;
(xiv) concentration of fibrinogen in plasma;
(xv) concentration of urea in plasma;
(xvi) concentration of glucose in serum;
(xvii) length of telomers in blood cells;
(xviii) concentration of triglycerides in serum;
(xix) concentration of immunoglobulin M (IgM) in serum;
(xx) concentration of adiponectin in serum;
(xxi) accumulated concentration of threonine and lactate in urine;

(xxii) concentration of homocysteine in plasma;
(xxiii) concentration of lycopene in plasma;
(xxiv) concentration of cholesterol in serum;
(xxv) concentration of glycosylated hemoglobin ($HbA_{1c}$) in whole blood;
(xxvi) concentration of glycine in urine;
(xxvii) number of T cell receptor excision circles (Trec) in 100,000 T cells from whole blood;
(xxviii) concentration of creatinine in urine.

3. The method according to claim 1, wherein the human being is male, in step (b1) one or more of the following biomarkers (xi) to (xxiii) are determined in addition to the biomarkers (i) to (x), and the necessary biological samples are provided in step (a):

(xi) cumulative level of cytosine methylation at gene positions FHL_2_CpG_19, and FHL_2_CpG_20 in blood cells;
(xii) number of T cell receptor excision circles (Trec) in 100,000 T cells from whole blood;
(xiii) decadic logarithm of the ratio between the concentration of NGA2F glycan in serum and the concentration of NA2F glycan in serum;
(xiv) concentration of prostate specific antigen (PSA) in serum;
(xv) concentration of fibrinogen in plasma;
(xvi) concentration of albumin in plasma;
(xvii) concentration of glucose in serum;
(xviii) concentration of NGA2FB glycan in serum;
(xix) ratio of the concentration of Cu in plasma to the concentration of Zn in plasma;
(xx) ratio of the number of $CD16^+CD56^+$ cells in whole blood to the number of $CD45^+$ cells in whole blood;
(xxi) concentration of IgG antibodies directed against *Clostridium tetani* in plasma;
(xxii) ratio of the number of $CD3^+CD8^+$ cells in whole blood to the number of $CD45^+$ cells in whole blood;
(xxiii) concentration of beta-hydroxyisovalerate in urine.

4. The method according to any one of claims 1 to 3, wherein in step (c) determining the biological age of the human being based on the biomarkers determined in step (b1)/(b2) comprises the steps:

(c1) establishing a multiple linear regression model for the determined biomarkers based on the data provided in the study underlying the present invention;
(c2) determining from the multiple linear regression model established in step (c1)

(i) the correlation coefficient $r^2$,
(ii) the weighting factors $b_i$ for each determined biomarker, wherein i is 1 through n, and n is the number of biomarkers determined, and
(iii) the intercept c;

(c3) calculating an apparent biological age B according to formula (1)

$$B = \sum_{i=1}^{n} b_i x_i + c \qquad (1)$$

wherein

$b_i$ are the weighting factors determined in step (c2),
$x_i$ are the values of the respective biomarkers as determined in step (b1)/(b2), wherein i is 1 to n and
c is the intercept determined in step (c2); and

(c4) calculating the corrected biological age (Bioage) according to formula (2)

$$Bioage = \frac{B - mean(a)}{r^2} + mean(a) \qquad (2)$$

wherein

B is the apparent biological age determined in step (c3),
and
mean(a) is the mean chronological age of the statistical population used in the study underlying the present invention.

5. The method according to claim 1, wherein the human being is female, the biomarkers (i) to (x) are determined in step (b1), and determining the biological age of the human being based on said biomarkers in step (c) comprises the steps of:

(c1) calculating an apparent biological age B according to formula (3)

$$B = \sum_{i=1}^{10} b_i x_i + c \qquad (3)$$

wherein

$b_1$ is a number between 40.37 and 49.33,
$x_1$ is the determined value of biomarker (i) expressed as a percentage between 0 and 1,
$b_2$ is a number between 42.86 and 52.37,
$x_2$ is the determined value of biomarker (ii) expressed as a percentage between 0 and 1,
$b_3$ is a number between -28.00 and -22.92,
$x_3$ is the determined value of biomarker (iii) expressed as a percentage between 0 and 1,
$b_4$ is a number between 26.52 and 32.40,
$x_4$ is the determined value of biomarker (iv) expressed as a percentage between 0 and 1,
$b_5$ is a number between 11.88 and 14.50,
$x_5$ is the determined value of biomarker (v) expressed as a percentage between 0 and 1,
$b_6$ is a number between -0.97 and -0.80,
$x_6$ is the determined value of biomarker (vi) expressed in $\mu$g per dL,
$b_7$ is a number between 0.023 and 0.027,
$x_7$ is the determined value of biomarker (vii) expressed in ng per mL,
$b_8$ is a number between 9.78 and 11.94
$x_8$ is the determined value of biomarker (viii),
$b_9$ is a number between 0.09 and 0.11
$x_9$ is the determined value of biomarker (ix) expressed in $\mu$mole per L,
$b_{10}$ is a number between 0.028 and 0.034,
$x_{10}$ is the determined value of biomarker (x) expressed in $\mu$mole per L, and
c is a number between -14.47 and -11.85; and

(c2) calculating the corrected biological age (Bioage) according to formula (2)

$$Bioage = \frac{B - mean(a)}{r^2} + mean(a) \qquad (2)$$

wherein

B is the apparent biological age determined in step (c1),

mean(a) is a number between 50.27 and 61.43, and
$r^2$ is a number between 0.79 and 0.95.

6. The method according to claim 5, wherein

$b_1$ is 44.85,
$b_2$ is 47.616,
$b_3$ is -25.457,
$b_4$ is 29.463,
$b_5$ is 13.19,
$b_6$ is -0.889,
$b_7$ is 0.025,
$b_8$ is 10.863,
$b_9$ is 0.1,
$b_{10}$ is 0.031,
c is -13.16
mean(a) is 55.85, and
$r^2$ is 0.87.

7. The method according to claim 1, wherein the human being is male, the biomarkers (i) to (x) are determined in step (b2), and determining the biological age of the human being based on said biomarkers in step (c) comprises the steps of:

(c1) calculating an apparent biological age B according to formula (3)

$$B = \sum_{i=1}^{10} b_i x_i + c \qquad (3)$$

wherein

$b_1$ is a number between 32.84 and 40.12,
$x_1$ is the determined value of biomarker (i) expressed as a percentage between 0 and 1,
$b_2$ is a number between 33.53 and 40.97,
$x_2$ is the determined value of biomarker (ii) expressed as a percentage between 0 and 1,
$b_3$ is a number between 42.52 and 51.95,
$x_3$ is the determined value of biomarker (iii) expressed as a percentage between 0 and 1,
$b_4$ is a number between 4.06 and 4.95,
$x_4$ is the determined value of biomarker (iv) expressed as a percentage between 0 and 1,
$b_5$ is a number between -0.18 and -0.16,
$x_5$ is the determined value of biomarker (v),
$b_6$ is a number between 1.39 and 1.69,
$x_6$ is the determined value of biomarker (vi),
$b_7$ is a number between -1.14 and -0.94,
$x_7$ is the determined value of biomarker (vii) expressed in g per dL,
$b_8$ is a number between 0.022 and 0.026,
$x_8$ is the determined value of biomarker (viii) expressed in mg per dL,
$b_9$ is a number between -3.44 and -2.82,
$x_9$ is the determined value of biomarker (ix) expressed in $\mu$mole per L,
$b_{10}$ is a number between 0.027 and 0.031,
$x_{10}$ is the determined value of biomarker (x) expressed in $\mu$mole per L, and
c is a number between -4.24 and -3.47; and

(c2) calculating the corrected biological age (Bioage) according to the formula (2)

$$Bioage = \frac{B - mean(a)}{r^2} + mean(a) \qquad (2)$$

wherein

B is the apparent biological age determined in step (c1),
mean(a) is a number between 50.27 and 61.43, and
$r^2$ is a number between 0.78 and 0.94.

8. The method according to claim 7, wherein

$b_1$ is 36.481,
$b_2$ is 37.25,
$b_3$ is 47.234,
$b_4$ is 4.506,
$b_5$ is -0.172,
$b_6$ is 1.538,
$b_7$ is -1.044,
$b_8$ is 0.024,
$b_9$ is -3.131,
$b_{10}$ is 0.029,
c is -3.858
mean(a) is 55.85, and
$r^2$ is 0.86.

9. The method according to any one of claims 1 to 8, wherein the human being has a chronological age of between 30 and 80 years.

10. The use of at least the following biomarkers (i) to (x) for the determination of the biological age of a female human being:

(i) cumulative level of cytosine methylation at gene positions ELOVL_2_CpG_11, ELOVL_2_CpG_12, ELOVL_2_CpG_13, and ELOVL_2_CpG_14 in blood cells;
(ii) cumulative level of cytosine methylation at gene positions ELOVL_2_CpG_15, ELOVL_2_CpG_16, and ELOVL_2_CpG_17 in blood cells;
(iii) cumulative level of cytosine methylation at gene positions FHL_2_CpG_11, and FHL_2_CpG_12 in blood cells;
(iv) cumulative level of cytosine methylation at gene positions FHL_2_CpG_13, FHL_2_CpG_14, and FHL_2_CpG_15 in blood cells;
(v) cumulative level of cytosine methylation at gene positions FHL_2_CpG_16 and FHL_2_CpG_17 in blood cells;
(vi) concentration of dehydroepiandrosterone sulfate in plasma;
(vii) concentration of ferritin in serum;
(viii) decadic logarithm of the ratio between the concentration of NGA2F glycan in serum and the concentration of NA2F glycan in serum;
(ix) concentration of alpha-tocopherol in plasma;
(x) concentration of cysteine in plasma.

11. The use according to claim 10, wherein one or more of the following biomarkers (xi) to (xxviii) are used in addition to the biomarkers (i) to (x):

(xi) cumulative level of cytosine methylation at gene positions ELOVL_2_CpG_22, ELOVL_2_CpG_23, and ELOVL_2_CpG_24 in blood cells;
(xii) cumulative level of cytosine methylation at gene positions FHL_2_CpG_19, and FHL_2_CpG_20 in blood cells;
(xiii) concentration of NGA2FB glycan in serum;
(xiv) concentration of fibrinogen in plasma;

(xv) concentration of urea in plasma;
(xvi) concentration of glucose in serum;
(xvii) length of telomers in blood cells;
(xviii) concentration of triglycerides in serum;
(xix) concentration of immunoglobulin M (IgM) in serum;
(xx) concentration of adiponectin in serum;
(xxi) accumulated concentration of threonine and lactate in urine;
(xxii) concentration of homocysteine in plasma;
(xxiii) concentration of lycopene in plasma;
(xxiv) concentration of cholesterol in serum;
(xxv) concentration of glycosylated hemoglobin ($HbA_{1c}$) in whole blood;
(xxvi) concentration of glycine in urine;
(xxvii) number of T cell receptor excision circles (Trec) in 100,000 T cells from whole blood;
(xxviii) concentration of creatinine in urine.

**12.** The use of at least the following biomarkers (i) to (x) for the determination of the biological age of a male human being:

(i) cumulative level of cytosine methylation at gene positions ELOVL_2_CpG_11, ELOVL_2_CpG_12, ELOVL_2_CpG_13, and ELOVL_2_CpG_14 in blood cells;
(ii) cumulative level of cytosine methylation at gene positions ELOVL_2_CpG_15, ELOVL_2_CpG_16, and ELOVL_2_CpG_17 in blood cells;
(iii) cumulative level of cytosine methylation at gene positions FHL_2_CpG_13, FHL_2_CpG_14, and FHL_2_CpG_15 in blood cells;
(iv) cumulative level of cytosine methylation at gene position FHL_2_CpG_18;
(v) concentration of creatinine in urine;
(vi) ratio of the number of $CD3^+CD4^+$ cells in whole blood to the number of $CD8^+$ cells in whole blood;
(vii) concentration of dehydroepiandrosterone sulfate in plasma;
(viii) concentration of alpha-2-macroglubulin in serum;
(ix) concentration of lycopene in plasma;
(x) concentration of cysteine in plasma.

**13.** The use according to claim 12, wherein one or more of the following biomarkers (xi) to (xxiii) are used in addition to the biomarkers (i) to (x):

(xi) cumulative level of cytosine methylation at gene positions FHL_2_CpG_19, and FHL_2_CpG_20 in blood cells;
(xii) number of T cell receptor excision circles (Trec) in 100,000 T cells from whole blood;
(xiii) decadic logarithm of the ratio between the concentration of NGA2F glycan in serum and the concentration of NA2F glycan in serum;
(xiv) concentration of prostate specific antigen (PSA) in serum;
(xv) concentration of fibrinogen in plasma;
(xvi) concentration of albumin in plasma;
(xvii) concentration of glucose in serum;
(xviii) concentration of NGA2FB glycan in serum;
(xix) ratio of the concentration of Cu in plasma to the concentration of Zn in plasma;
(xx) ratio of the number of $CD16^+CD56^+$ cells in whole blood to the number of $CD45^+$ cells in whole blood;
(xxi) concentration of antibodies directed against *Clostridium tetani* in plasma;
(xxii) ratio of the number of $CD3^+CD8^+$ cells in whole blood to the number of $CD45^+$ cells in whole blood;
(xxiii) concentration of beta-hydroxyisovalerate in urine.

**14.** The use according to claim 10 or claim 12, wherein only the biomarkers (i) to (x) are used.

Figure 1

Figure 2

28

Figure 3

Figure 4

Figure 5

Figure 6

Figure 7

Figure 8

dehydroepiandrosteron sulfate [μg/dl]

RASIG male = 1066
y = -0.12x + 11
rp= -0.484 rnp= -0.547

RASIG female = 1139
y = -0.07x + 6.55
rp= -0.431 rnp= -0.48

p=1.0% quantile hidden

p=1.0% quantile hidden

Figure 9

Figure 11

Figure 12

Figure 13

Figure 14

Figure 15.

Figure 16

42

Figure 17

Figure 18

Figure 19

# Serum glucose [mmol/l]

**RASIG  male =  985**

y = 0.02x + 4.12

rp = 0.205  rnp = 0.246

**RASIG  female =  1095**

y = 0.02x + 3.83

rp = 0.231  rnp = 0.313

p=1.0% quantile hidden

p=1.0% quantile hidden

Figure 20

Figure 21

Figure 22

Figure 23

Figure 24

Figure 25

Figure 26

Figure 27

53

Figure 28

Figure 29

Figure 30

Figure 31

Figure 32

Figure 33

CD16+CD56+/CD45 +

Figure 34

Tetanus IgG antibodies

Figure 35

61

Figure 36

Figure 37

CD3+CD4+/CD8+

Figure 38

EP 2 781 602 A1

64

Figure 39

Europäisches
Patentamt

European
Patent Office

Office européen
des brevets

**EUROPEAN SEARCH REPORT**

Application Number

EP 13 00 1450

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | D. G. HERNANDEZ ET AL: "Distinct DNA methylation changes highly correlated with chronological age in the human brain", HUMAN MOLECULAR GENETICS, vol. 20, no. 6, 7 January 2011 (2011-01-07), pages 1164-1172, XP55069768, ISSN: 0964-6906, DOI: 10.1093/hmg/ddq561 * the whole document * | 1-14 | INV. C12Q1/68 G06F19/00 |
| X | PAOLO GARAGNANI ET AL: "Methylation of ELOVL 2 gene as a new epigenetic marker of age", AGING CELL, vol. 11, no. 6, 14 October 2012 (2012-10-14), pages 1132-1134, XP55069767, ISSN: 1474-9718, DOI: 10.1111/acel.12005 * the whole document * | 1-14 | |
| X | MADRIGANO JAIME ET AL: "Aging and epigenetics Longitudinal changes in gene-specific DNA methylation", EPIGENETICS, LANDES BIOSCIENCE, US, vol. 7, no. 1, 1 January 2012 (2012-01-01), pages 63-70, XP008157957, ISSN: 1559-2294, DOI: 10.4161/EPI.7.1.18750 * the whole document * | 1-14 | TECHNICAL FIELDS SEARCHED (IPC) C12Q G06F |
| X | BOCKLANDT S ET AL: "Epigenetic Predictor of Age", PLOS ONE, PUBLIC LIBRARY OF SCIENCE, US, vol. 6, no. 6, 22 June 2011 (2011-06-22), pages E14821-1, XP002687317, ISSN: 1932-6203, DOI: 10.1371/JOURNAL.PONE.0014821 * the whole document * | 1-14 | |

-/--

The present search report has been drawn up for all claims

| Place of search | Date of completion of the search | Examiner |
|---|---|---|
| Munich | 5 July 2013 | Mueller, Frank |

EPO FORM 1503 03.82 (P04C01)

Europäisches Patentamt
European Patent Office
Office européen des brevets

**EUROPEAN SEARCH REPORT**

Application Number

EP 13 00 1450

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KOCH CARMEN M ET AL: "Epigenetic-aging-signature to determine age in different tissues", AGING, NEW YORK, NY, US, vol. 3, no. 10, 1 October 2011 (2011-10-01), pages 1018-1027, XP002687316, ISSN: 0160-2721 * the whole document * | 1-14 | |
| X | WO 02/10445 A2 (EPIGENOMICS AG [DE]; OLEK ALEXANDER [DE]) 7 February 2002 (2002-02-07) * the whole document * | 1-14 | |
| X | WO 2012/162139 A1 (UNIV CALIFORNIA [US]; VILAIN ERIC [US]; BOCKLANDT SVEN [US]; HORVATH S) 29 November 2012 (2012-11-29) * the whole document * | 1-14 | |
| X | J. P. DE MAGALHAES ET AL: "Meta-analysis of age-related gene expression profiles identifies common signatures of aging", BIOINFORMATICS, vol. 25, no. 7, 2 February 2009 (2009-02-02), pages 875-881, XP055069769, ISSN: 1367-4803, DOI: 10.1093/bioinformatics/btp073 * the whole document * | 1-14 | TECHNICAL FIELDS SEARCHED (IPC) |

The present search report has been drawn up for all claims

| Place of search | Date of completion of the search | Examiner |
|---|---|---|
| Munich | 5 July 2013 | Mueller, Frank |

## ANNEX TO THE EUROPEAN SEARCH REPORT
## ON EUROPEAN PATENT APPLICATION NO.

EP 13 00 1450

This annex lists the patent family members relating to the patent documents cited in the above-mentioned European search report.
The members are as contained in the European Patent Office EDP file on
The European Patent Office is in no way liable for these particulars which are merely given for the purpose of information.

05-07-2013

| Patent document cited in search report | | Publication date | Patent family member(s) | | Publication date |
|---|---|---|---|---|---|
| WO 0210445 | A2 | 07-02-2002 | AT | 330037 T | 15-07-2006 |
| | | | AU | 8954701 A | 13-02-2002 |
| | | | CA | 2423849 A1 | 27-03-2003 |
| | | | DE | 10038733 A1 | 21-02-2002 |
| | | | EP | 1320632 A2 | 25-06-2003 |
| | | | JP | 2004504855 A | 19-02-2004 |
| | | | US | 2004132026 A1 | 08-07-2004 |
| | | | WO | 0210445 A2 | 07-02-2002 |
| WO 2012162139 | A1 | 29-11-2012 | NONE | | |

EPO FORM P0459

For more details about this annex : see Official Journal of the European Patent Office, No. 12/82